# EUROPEAN PATENT APPLICATION

(11) **EP 4 477 236 A1**
(43) Date of publication of application: **18.12.2024**
(21) Application number: 23305950.0
(22) Date of filing: 14.06.2023
(51) Int. Cl.: A61K 47/68, A61K 45/06, A61P 35/00

(54) **COMBINATION THERAPY FOR TREATING A TUMOR USING ADC COMPRISING ANTI-CD71 ANTIBODIES AND BH3 MIMETICS**

(71) Applicant: INATHERYS, 91058 Evry Cedex (FR)
(72) Inventor: LAUNAY, Pierre, RUEIL MALMAISON (FR); SOUCHET, Hervé, 75020 PARIS (FR); STUBBS, Elisa, 92120 MONTROUGE (FR)
(74) Representative: Plasseraud IP

(57) **Abstract**

The present invention relates to the use of an antibody-drug conjugate of the formula (I): Ab-[L'-D]p (I), wherein:
- Ab is an anti-Transferrin Receptor (TfR) antibody which binds specifically to SEQ ID NO:16,
- L' is a cleavable or a non-cleavable linker moiety covalently bonded to said antibody,
- D is a cytotoxic drug moiety covalently bonded to L',
- p is an integer from 1 to 8, preferably 4,
for use in the treatment of a tumor, characterized in that said antibody-drug conjugate is administered in combination with at least one BH3 mimetics compound.

## Description

### Technical field

It is hereinafter disclosed a combination therapy for the treatment of cancer, using antibody-drug conjugate (ADC) and a BH3 mimetics. In said antibody-drug conjugates, the antibody specifically binds to TfR, the transferrin receptor, and the drug is preferably chosen among a cytotoxic drug. Said BH3 mimetics is preferably venetoclax. The combination therapy is useful in particular in treating proliferative diseases including solid and blood cancers, such as lymphoma or leukaemia.

### Background

The use of antibodies and ADC for the treatment of cancer is more and more developed. These antibodies can be used alone or in combination. Antibody-based therapies of human cancer have notably become first line treatments in certain settings.

The transferrin receptor (CD71) (hereafter referred as "TfR") is also a key target for the treatment of cancer. TfR is a disulfide-linked homodimeric transmembrane glycoprotein consisting of two 760-amino acid monomers of approximately 90 kDa each. TfR plays a crucial role in the regulation of iron uptake and cell growth. When diferric transferrin binds to its cell surface receptor, it is internalized via clathrin-coated pits to acidic vesicles where the iron-transferrin complex is dissociated. After release, the receptor and apo-transferrin recycle back to the cell surface.

TfR is constitutively expressed at the cell plasma membrane of tissues that are constantly renewed, such as precursors of blood cells in the bone marrow, hepatocytes in the liver, keratinocytes in the epidermis and enterocytes in crypts of intestinal epithelium.

Several studies have shown that TfR is expressed more abundantly in malignant tissues than in their healthy counterparts (Gatter et al., J Clin Pathol., 36,539-545, 1983 - Faulk et al., Lancet., 2,390-392, 1980 - Shindelman et al., Int J Cancer, 27,329-334, 1981). Several authors have reported therapeutic approaches based on this idea using anti-TfR antibodies or transferrin itself conjugated to drugs to kill malignant cells. It has also been proposed to use anti-TfR antibodies to block the interaction between transferrin and TfR, and consequently preventing iron uptake, leading to iron deprivation and negative regulation of cell growth. However, although many publications describe the generation of anti-TfR antibodies, there are very few reports of anti-TfR monoclonal antibodies (mAbs) having an antiproliferative activity.

In a previous publication (Moura et al., J Exp Med, 194, 417-425, 2001), the authors have reported a mouse monoclonal IgG (IgG2kappa), designated A24, that bind to the human TfR.

WO2005/111082 discloses the A24 antibody, a murine antibody able to block activated T cell proliferation, and which appeared to be more efficient than the previously described mAb 42/6 in inhibiting proliferation of T cells. A24 also reduced TfR expression at the cell surface and impaired TfR recycling. A24 is also able to block the *ex vivo* proliferation of malignant T cells from both acute and chronic forms of ATL (Moura et al., Blood, 103,5, 1838-45,1 March 2004, Callens et al., 2010; J. Exp. Med., Vol 207 No 4, pp731-750). This antibody has been also described as able to prevent the mantle cell lymphoma tumor development both *in vitro* and *in vivo* (Lepelletier et al. Cancer Res 2007; 67:1145-1154; Callens et al. 2008, Leukemia, 22, 42-48).

WO2017/013230 further discloses humanized versions of mouse A24 antibodies, and their use in treating proliferative disorders. It also mentions an anti-TfR antibody conjugated to a therapeutic moiety, although no specific ADC is disclosed.

WO2019/197428 further discloses ADC comprising anti-TfR antibody.

Combination treatments with BH3-mimetics and an immunotoxin are notably disclosed in Abid R. Mattoo and David J. Fitzgerald (Int. J. Cancer: 132, 978-987 (2013) 2012 UICC) or Traini *et al.* (doi: 10.1158/1535-7163.MCT-10-0257). Said immunotoxin is for example HB21-PE40, i.e. an immunotoxin directed at the human transferrin receptor containing Pseudomonas exotoxin A (Batra JK,Fitzgerald DJ,Chaudhary VK, et al. Single-chain immunotoxins directed at the human transferrin receptor containing Pseudomonas exotoxin A or diphtheria toxin: anti-TFR(Fv)-PE40 and DT388-anti-TFR(Fv). Mol Cell Biol 1991 ;11 :2200-5). US2012/0276190 also refers to therapy with a chimeric molecule and a pro-apoptotic agent; wherein said chimeric molecule can comprise an antibody against a cell surface antigen (CD71 being cited among other cell surface antigens).

Nevertheless, there is always a need for new therapy highly efficient, with high specificity and low toxicity.

### Summary

The disclosure thus relates to an antibody-drug conjugate of the formula (I): Ab-[L'-D]p (I), wherein:
- Ab is an anti-Transferrin Receptor (TfR) antibody which binds specifically to SEQ ID NO:16,
- L' is a cleavable or a non-cleavable linker moiety covalently bonded to said antibody, preferably a cleavable linker,
- D is a cytotoxic drug moiety covalently bonded to L',
- p is an integer from 1 to 8, preferably 4,
for use in the treatment of a tumor, characterized in that said antibody-drug conjugate is administered in combination with at least one BH3 mimetics compound.

The disclosure also relates to a BH3 mimetics compound for use in the treatment of a tumor, characterized in that said BH3 mimetics compound is administered in combination with at least an antibody-drug conjugate of the formula (I): Ab-[L'-D]p (I), wherein:
- Ab is an anti-Transferrin Receptor (TfR) antibody which binds specifically to SEQ ID NO:16,
- L' is a cleavable or a non-cleavable linker moiety covalently bonded to said antibody,
- D is a cytotoxic drug moiety covalently bonded to L',
- p is an integer from 1 to 8, preferably 4.

The disclosure also relates to a composition comprising:
(a) an antibody-drug conjugate of the formula (I): Ab-[L'-D]p (I), wherein:
   - Ab is an anti-Transferrin Receptor (TfR) antibody which binds specifically to SEQ ID NO:16,
   - L' is a cleavable or a non-cleavable linker moiety covalently bonded to said antibody,
   - D is a cytotoxic drug moiety covalently bonded to L',
   - p is an integer from 1 to 8, preferably 4,
(b) a BH3 mimetics, and
(c) one or more pharmaceutical acceptable excipient, diluent or carrier.

The disclosure also relates to a combination product comprising:
(a) an antibody-drug conjugate of the formula (I): Ab-[L'-D]p (I), wherein:
   - Ab is an anti-Transferrin Receptor (TfR) antibody which binds specifically to SEQ ID NO:16,
   - L' is a cleavable or a non-cleavable linker moiety covalently bonded to said antibody,
   - D is a cytotoxic drug moiety covalently bonded to L',
   - p is an integer from 1 to 8, preferably 4, and
(b) a BH3 mimetics.

The disclosure further relates to the use of an antibody-drug conjugate of the formula (I): Ab-[L'-D]p (I), wherein:
- Ab is an anti-Transferrin Receptor (TfR) antibody which binds specifically to SEQ ID NO:16,
- L' is a cleavable or a non-cleavable linker moiety covalently bonded to said antibody,
- D is a cytotoxic drug moiety covalently bonded to L',
- p is an integer from 1 to 8, preferably 4,
for the manufacture of a medicament for the treatment of tumor, in combination with a BH3 mimetics.

The disclosure also relates to a method for the treatment of a tumor, comprising the steps of:
- administration of a therapeutically efficient amount of an antibody-drug conjugate of the formula (I): Ab-[L'-D]p (I), wherein:
   ∘ Ab is an anti-Transferrin Receptor (TfR) antibody which binds specifically to SEQ ID NO:16,
   ∘ L' is a cleavable or a non-cleavable linker moiety covalently bonded to said antibody,
   ∘ D is a cytotoxic drug moiety covalently bonded to L',
   ∘ p is an integer from 1 to 8, preferably 4,
- administration of a therapeutically efficient amount of a BH3 mimetics.

The disclosure also relates to the use of a BH3 mimetics for the manufacture of a medicament for the treatment of tumor, in combination with an antibody-drug conjugate of the formula (I): Ab-[L'-D]p (I), wherein:
- Ab is an anti-Transferrin Receptor (TfR) antibody which binds specifically to SEQ ID NO:16,
- L' is a cleavable or a non-cleavable linker moiety covalently bonded to said antibody,
- D is a cytotoxic drug moiety covalently bonded to L',
- p is an integer from 1 to 8, preferably 4.

The disclosure also relates to an antibody-drug conjugate of the formula (II): Ab-L-Z-X-D, wherein:
- Ab is an anti-TfR antibody which binds specifically to SEQ ID NO:16,
- L is a linker molecule bonded to said antibody, said linker molecule being of formula (III): 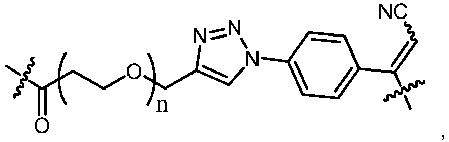 wherein n is an integer comprised between 2 and 20,
- Z is a dipeptide of valine-citrulline bonded to L,
- X is an aminobenzyl ester self-immolative group bounded to Z,
- D is a drug bounded to X,
- p is an integer from 1 to 8, preferably 4,
for use in the treatment of a tumor, characterized in that said antibody-drug conjugate is administered in combination with at least one BH3 mimetics compound.

In a specific embodiment, which can be combined with other embodiments herein disclosed, said antibody-drug conjugate is of the formula (II): Ab-L-Z-X-D, wherein:
- Ab is an anti-TfR antibody,
- L is a linker molecule bonded to said antibody, said linker molecule being of formula (III): 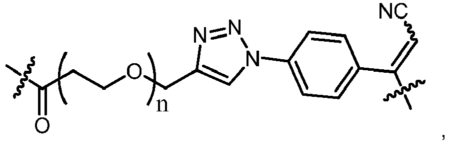 wherein n is an integer comprised between 2 and 20,
- Z is a dipeptide of valine-citrulline bonded to L,
- X is an aminobenzyl ester self-immolative group bounded to Z,
- D is a drug bounded to X.

In a specific embodiment, which can be combined with other embodiments herein disclosed, said BH3 mimetics is chosen among: venetoclax, navitoclax, obatoclax and S64315, preferably venetoclax, navitoclax, obatoclax, more preferably venetoclax.

In a specific embodiment, which can be combined with other embodiments herein disclosed, the drug is a cytotoxic drug, notably monomethyl auristatin E.

In a specific embodiment, which can be combined with other embodiments herein disclosed, X is a para-aminobenzyl ester group covalently bounded to Z, said X being of the following formula (IV): 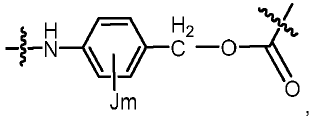 J being an optional substituent chosen among F, Cl, Br, NO₂, NHCOCH₃, N(CH₃)₂, NHCOCF₃, alkyl, and haloalkyl, and m being an integer of 0, 1, 2, 3 and 4, said X being preferably a para-aminobenzyl ester wherein m is 0.

In a specific embodiment, which can be combined with other embodiments herein disclosed, L is covalently bonded to one or more thiol residues of said antibody, preferably, said L corresponding to a linker molecule of formula (V):

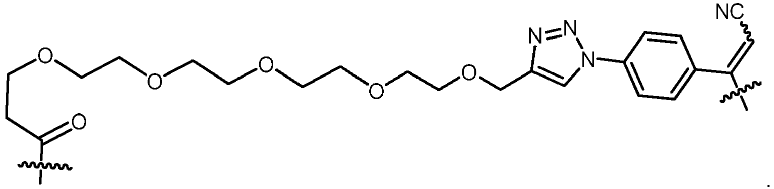

In a specific embodiment, which can be combined with other embodiments herein disclosed, said antibody includes full-length antibodies or antibodies fragments containing antigen binding portions, preferably full-length antibodies.

In a specific embodiment, which can be combined with other embodiments herein disclosed, said antibody binds to the transferrin receptor with a K_{D} of 10nM or less, preferably with a K_{D} of 1 nM or less.

In a specific embodiment, which can be combined with other embodiments herein disclosed, said antibody is a monoclonal antibody and chimeric or a humanized antibody, preferably humanized.

In a specific embodiment, which can be combined with other embodiments herein disclosed, (a) said antibody comprises a human IgG4 isotype constant region, or a mutant or chemically modified constant region, wherein said mutant or chemically modified constant region confers no or decreased ADCC activity to said antibody when compared to a corresponding antibody with wild type IgG1 isotype constant region, or (b) said antibody comprises a human IgG1 isotype constant region, or a mutant or chemically modified constant region, wherein said mutant or chemically modified constant region confers increased ADCC activity said antibody when compared to a corresponding antibody with wild type IgG1 isotype constant region, or (c) antibody fragments such as F(ab)'₂, Fab, scFv, ...

In a specific embodiment, which can be combined with other embodiments herein disclosed, said antibody or antibody fragment is divalent. Antibody fragments are preferably divalent fragments.

In a specific and preferred embodiment, which can be combined with other embodiments herein disclosed, said anti-TfR antibody comprises either:
(a) a variable heavy chain polypeptide comprising HCDR1 of SEQ ID NO:1, HCDR2 of SEQ ID NO:2, HCDR3 of SEQ ID NO:3 and a variable light chain polypeptide comprising LCDR1 of SEQ ID NO:4, LCDR2 of SEQ ID NO:5 and LCDR3 of SEQ ID NO:6;
(b) a variable heavy chain polypeptide comprising HCDR1 of SEQ ID NO:1, HCDR2 of SEQ ID NO:2, HCDR3 of SEQ ID NO:3 and a variable light chain polypeptide comprising LCDR1 of SEQ ID NO:4, LCDR2 of SEQ ID NO:8 and LCDR3 of SEQ ID NO:6;
(c) a variable heavy chain polypeptide comprising VH of SEQ ID NO:11 and a variable light chain polypeptide comprising VL of SEQ ID NO:13;
(d) a variable heavy chain polypeptide comprising VH of SEQ ID NO:11 and a variable light chain polypeptide comprising VL of SEQ ID NO:14;
(e) a variable heavy chain polypeptide comprising VH of SEQ ID NO:11 and a variable light chain polypeptide comprising VL of SEQ ID NO:15;
(f) a variable heavy chain polypeptide comprising VH of SEQ ID NO:12 and a variable light chain polypeptide comprising VL of SEQ ID NO:13;
(g) a variable heavy chain polypeptide comprising VH of SEQ ID NO:12 and a variable light chain polypeptide comprising VL of SEQ ID NO:14;
(h) a variable heavy chain polypeptide comprising VH of SEQ ID NO:12 and a variable light chain polypeptide comprising VL of SEQ ID NO:15,
   preferably said antibody is mAb1 comprising heavy chain of SEQ ID NO:18 and light chain of SEQ ID NO:17.

In a specific and preferred embodiment, which can be combined with other embodiments herein disclosed, said antibody-drug conjugate is of the formula (II): Ab-L-Z-X-D, wherein:
- Ab is an antibody comprising heavy chain of SEQ ID NO:18 and light chain of SEQ ID NO:17,
- L is a linker molecule of formula (V),
- Z is a dipeptide of valine-citrulline,
- X is a para-aminobenzyl ester of formula (IV) wherein m is 0,
- D represents monomethyl auristatin E.

In a specific and preferred embodiment, which can be combined with other embodiments herein disclosed, *said antibody-drug conjugate is of the formula (II): Ab-L-Z-X-D, wherein:
- Ab is an antibody comprising heavy chain of SEQ ID NO:18 and light chain of SEQ ID NO:17,
- L is a linker molecule of formula (V),
- Z is a dipeptide of valine-citrulline,
- X is a para-aminobenzyl ester of formula (IV) wherein m is 0,
- D represents monomethyl auristatin E, and * said BH3 mimetics is venetoclax.

In a specific and preferred embodiment, which can be combined with other embodiments herein disclosed, *said antibody-drug conjugate is of the formula (II): Ab-L-Z-X-D, wherein:
- Ab is an antibody comprising heavy chain of SEQ ID NO:18 and light chain of SEQ ID NO:17,
- L is a linker molecule of formula (V),
- Z is a dipeptide of valine-citrulline,
- X is a para-aminobenzyl ester of formula (IV) wherein m is 0,
- D represents monomethyl auristatin E, and *wherein BH3 mimetics is navitoclax.

In a specific and preferred embodiment, which can be combined with other embodiments herein disclosed, *said antibody-drug conjugate is of the formula (II): Ab-L-Z-X-D, wherein:
- Ab is an antibody comprising heavy chain of SEQ ID NO:18 and light chain of SEQ ID NO:17,
- L is a linker molecule of formula (V),
- Z is a dipeptide of valine-citrulline,
- X is a para-aminobenzyl ester of formula (IV) wherein m is 0,
- D represents monomethyl auristatin E, and *wherein BH3 mimetics is obatoclax.

In a specific and preferred embodiment, which can be combined with other embodiments herein disclosed, said tumor is a solid tumor or a hematologic tumor, and more specifically a lymphoma or leukemia.

In a specific and preferred embodiment, which can be combined with other embodiments herein disclosed, said tumor is venetoclax-resistant lymphoma or venetoclax-resistant leukemia.

In a specific embodiment, which can be combined with other embodiments herein disclosed, said antibody-drug conjugate and BH3 mimetics are administered simultaneous, separate or spread over time.

In a specific embodiment, which can be combined with other embodiments herein disclosed, said antibody-drug conjugate is administered intravenously and said BH3 mimetics is administered orally.

In a specific embodiment, which can be combined with other embodiments, the disclosure relates to a BH3 mimetics compound for use in the treatment of a tumor, in combination with an antibody-drug conjugate, wherein said antibody-drug conjugate is of the formula (II): Ab-L-Z-X-D, wherein:
- Ab is an anti-TfR antibody,
- L is a linker molecule bonded to said antibody, said linker molecule being of formula (III): 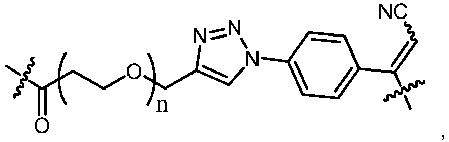 wherein n is an integer comprised between 2 and 20,
- Z is a dipeptide of valine-citrulline bonded to L,
- X is an aminobenzyl ester self-immolative group bonded to Z,
- D is a drug bonded to X.

In a specific embodiment, which can be combined with other embodiments, the disclosure relates to a composition wherein said antibody-drug conjugate is of the formula (II): Ab-L-Z-X-D, wherein:
- Ab is an anti-TfR antibody,
- L is a linker molecule bonded to said antibody, said linker molecule being of formula (III): 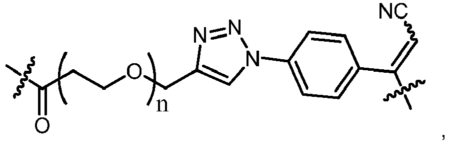 wherein n is an integer comprised between 2 and 20,
- Z is a dipeptide of valine-citrulline bonded to L,
- X is an aminobenzyl ester self-immolative group bonded to Z,
- D is a drug bonded to X,

In a specific embodiment which can be combined with other embodiments, said composition further comprises other active ingredients.

In a specific embodiment, which can be combined with other embodiments, the disclosure relates to a combination product, wherein said antibody-drug conjugate is of the formula (II): Ab-L-Z-X-D, wherein:
- Ab is an anti-TfR antibody,
- L is a linker molecule bonded to said antibody, said linker molecule being of formula (III): 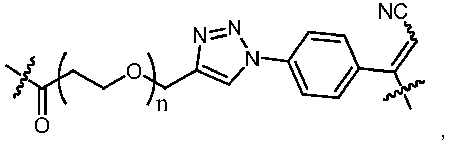 wherein n is an integer comprised between 2 and 20,
- Z is a dipeptide of valine-citrulline bonded to L,
- X is an aminobenzyl ester self-immolative group bonded to Z,
- D is a drug bonded to X.

In a specific embodiment which can be combined with other embodiments, said composition or combination product is disclosed for use in the treatment of a tumor.

In a specific embodiment which can be combined with other embodiments, it is herein disclosed a method for the treatment of a tumor wherein said antibody-drug conjugate is of the formula (II): Ab-L-Z-X-D, wherein:
- Ab is an anti-TfR antibody,
- L is a linker molecule bonded to said antibody, said linker molecule being of formula (III): 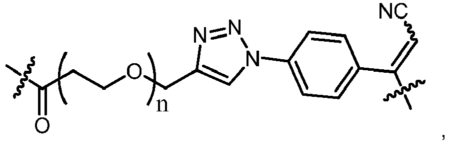 wherein n is an integer comprised between 2 and 20,
- Z is a dipeptide of valine-citrulline bonded to L,
- X is an aminobenzyl ester self-immolative group bonded to Z,
- D is a drug bonded to X.

In a specific embodiment which can be combined with other embodiments, it is herein disclosed a method for the treatment of a tumor, wherein said antibody-drug conjugate is of the formula (II): Ab-L-Z-X-D, wherein:
- Ab is an anti-TfR antibody,
- L is a linker molecule bonded to said antibody, said linker molecule being of formula (III): 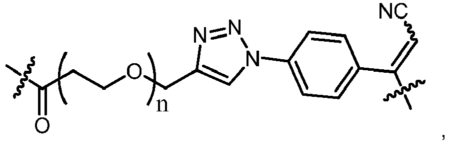 wherein n is an integer comprised between 2 and 20,
- Z is a dipeptide of valine-citrulline bonded to L,
- X is an aminobenzyl ester self-immolative group bonded to Z,
- D is a drug bonded to X, and wherein said BH3 mimetics is venetoclax.

In a specific embodiment which can be combined with other embodiments, it is herein disclosed a method for the treatment of a tumor, wherein said antibody-drug conjugate is of the formula (II): Ab-L-Z-X-D, wherein:
- Ab is an anti-TfR antibody,
- L is a linker molecule bonded to said antibody, said linker molecule being of formula (III): 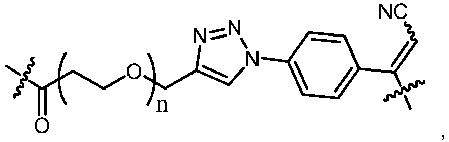 wherein n is an integer comprised between 2 and 20,
- Z is a dipeptide of valine-citrulline bonded to L,
- X is an aminobenzyl ester self-immolative group bonded to Z,
- D is a drug bonded to X, and wherein said BH3 mimetics is navitoclax.

In a specific embodiment which can be combined with other embodiments, it is herein disclosed a method for the treatment of a tumor, wherein said antibody-drug conjugate is of the formula (II): Ab-L-Z-X-D, wherein:
- Ab is an anti-TfR antibody,
- L is a linker molecule bonded to said antibody, said linker molecule being of formula (III): 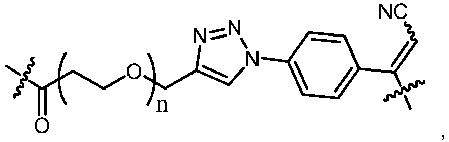 wherein n is an integer comprised between 2 and 20,
- Z is a dipeptide of valine-citrulline bonded to L,
- X is an aminobenzyl ester self-immolative group bonded to Z,
- D is a drug bonded to X, and wherein said BH3 mimetics is obatoclax.

In a specific embodiment which can be combined with other embodiments, it is herein disclosed a method for the treatment of a tumor, wherein said antibody-drug conjugate and BH3 mimetics are administered simultaneous, separate or spread over time.

In a specific embodiment which can be combined with other embodiments, it is herein disclosed the use of an antibody-drug conjugate, wherein said antibody-drug conjugate is of the formula (II): Ab-L-Z-X-D, wherein:
- Ab is an anti-TfR antibody,
- L is a linker molecule bonded to said antibody, said linker molecule being of formula (III): 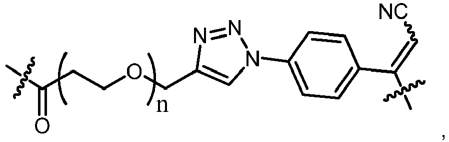 wherein n is an integer comprised between 2 and 20,
- Z is a dipeptide of valine-citrulline bonded to L,
- X is an aminobenzyl ester self-immolative group bonded to Z,
- D is a drug bonded to X.

In a specific embodiment which can be combined with other embodiments, it is herein disclosed the use of a BH3 mimetics for the manufacture of a medicament for the treatment of tumor, in combination with an antibody-drug conjugate of the formula (II): Ab-L-Z-X-D, wherein:
- Ab is an anti-TfR antibody,
- L is a linker molecule bonded to said antibody, said linker molecule being of formula (III): 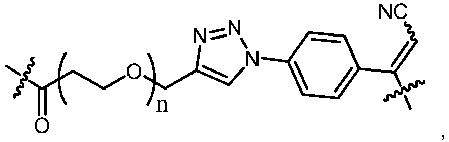 wherein n is an integer comprised between 2 and 20,
- Z is a dipeptide of valine-citrulline bonded to L,
- X is an aminobenzyl ester self-immolative group bonded to Z,
- D is a drug bonded to X.

### Legends of the Figures

**Figure 1****:** Combination effect on cellular proliferation of INA03 and Venetoclax. The AML cell line THP-1 (resistant to Venetoclax) was treated by several doses of Venetoclax alone or in combination with INA03 (at 0.25 or 0.5 ug/mL).
**Figure 2****:** Combination effect on cellular proliferation of INA03 and Venetoclax. The AML cell line HL-60 (sensitive to Venetoclax) was treated by several doses of Venetoclax alone or in combination with INA03 (at 0.1 ug/mL).
**Figure 3****:** Combination effect on cellular proliferation of INA03 and Venetoclax. The Mantle cell lymphoma line Mino was treated by several doses of Venetoclax alone or in combination with INA03 (at 0.1 ug/mL).
**Figure 4****:** Combination effect on cellular proliferation of INA03 and Venetoclax. The CLL cell line OSU was treated by several doses of Venetoclax alone or in combination with INA03 (at 0.1 ug/mL).
**Figures 5****&6:** Combination effect of INA03 and Venetoclax on apoptosis of primary AML cells. AML cells were treated with Venetoclax alone or in combination with INA03 for 96 h.
**Figure 7****:** Time course of proliferation on THP-1 cell in the presence of INA03 alone, Venetoclax alone or in combination.
**Figure 8****:** Time course of apoptosis on THP-1 cell in the presence of INA03 alone, Venetoclax alone or in combination. Apoptosis has been quantified as the number of green fluorescent caspase-3/7 active objects for each time point.
**Figure 9****:** Percentage of cells with depolarized mitochondria. THP-1 cells were incubated for 24h in medium culture alone (ctrl) or in the presence of INA03 (1 ug/mL), Venetoclax (200 nM) or a combination of both at the same dose.
**Figures 10****&11:** Percentage of apoptosis on activated and non-activated T cells. Activated (Figure 10) and non-activated (Figure 11) T cells were incubated for 96h in the presence of INA03 and Venetoclax alone or in combination, at various concentrations.
**Figure 12****:** Combination effect on cellular proliferation of INA03 and Navitoclax. The AML cell line THP-1 was treated by several doses of Navitoclax alone or in combination with INA03 (at 0.25 or 0.5 ug/mL).
**Figure 13****:** Combination effect on cellular proliferation of INA03 and Navitoclax. The AML cell line THP-1 was treated by several doses of Obatoclax alone or in combination with INA03 (at 0.25 or 0.5 ug/mL).
**Figure 14****:** *In vivo* efficacy of INA03 in combination with Venetoclax. Mice were treated with Venetoclax alone, INA03 alone or a combination of Venetoclax with INA03.

INA03 mentioned in said Figures refers to an antibody-drug conjugate of the formula (II): Ab-L-Z-X-D, wherein: Ab is an antibody comprising heavy chain of SEQ ID NO:18 and light chain of SEQ ID NO:17, L is a linker molecule of formula (V), Z is a dipeptide of valine-citrulline, X is a para-aminobenzyl ester of formula (IV) wherein m is 0, and D represents monomethyl auristatin E. Concentration unit referred to can be equally indicated "ug/mL" or "µg/mL".

### Detailed description

### Definitions

In order that the present disclosure may be more readily understood, certain terms are first defined. Additional definitions are set forth throughout the detailed description.

The term "CD71" or "transferrin receptor" or "TfR" refers to human TfR as defined in SEQ ID NO: 16, unless otherwise described. This sequence corresponds to Transferrin receptor protein 1 isoform 1 (homo sapiens) (NCBI Reference Sequence NP_003225.2).

The term "antibody" as referred to herein includes whole antibodies and any antigen binding fragments (i.e. "antigen-binding portion") or single chains thereof. A naturally occurring "antibody" is a glycoprotein comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (*e.g*. effector cells) and the first component (Clq) of the classical complement system. The term "antigen-binding portion" of an antibody (or simply "antigen portion"), as used herein, refers to full length or one or more fragments of an antibody that retain the ability to specifically bind to an antigen (e.g. a portion of TfR). It has been shown that the antigen-binding function of an antibody can be performed by fragments of a full-length antibody. Examples of binding fragments encompassed within the term "antigen-binding portion" of an antibody include a Fab fragment, a monovalent fragment consisting of the VL, VH, CL and CH1 domains; a F(ab)2 fragment; a bivalent fragment comprising two Fab fragments linked by a disulfide bridge at the hinge region; a Fd fragment consisting of the VH and CH1 domains; a Fv fragment consisting of the VL and VH domains of a single arm of an antibody; a UniBody consisting of a single arm with a modified IgG heavy chain, for example IgG4, at the hinge region, a domain antibody fragment (Ward et al., 1989 Nature 341:544-546), or a nanobody fragment which consists of a VH domain; and an isolated complementarity determining region (CDR); or any fusion proteins comprising such antigen-binding portion. Furthermore, although the two domains of the Fv fragment, VL and VH, are coded for by separate genes, they can be joined, using recombinant methods, by a synthetic linker that enables them to be made as a single chain protein in which the VL and VH regions pair to form monovalent molecules (known as single chain Fv (scFv); see e.g., Bird et al., 1988 Science 242:423-426; and Huston et al., 1988 Proc. Natl. Acad. Sci. 85:5879-5883). Such single chain antibodies are also intended to be encompassed within the term "antigen binding portion" of an antibody. These antibody fragments are obtained using conventional techniques known to those of skill in the art, and the fragments are screened for utility in the same manner as are intact antibodies. An "isolated antibody", as used herein, refers to an antibody that is substantially free of other antibodies having different antigenic specificities (*e.g.* an isolated antibody that specifically binds to TfR is substantially free of antibodies that specifically bind to other antigens than TfR). An isolated antibody that specifically binds to TfR may, however, have cross-reactivity to other antigens, such as TfR molecules from other species. Moreover, an isolated antibody may be substantially free of other cellular material and/or chemicals. The phrases "an antibody recognizing an antigen" and "an antibody specific for an antigen" are used interchangeably herein with the term "an antibody which binds specifically to an antigen".

The terms "monoclonal antibody" or "monoclonal antibody composition" as used herein refers to a preparation of antibody molecules of single molecular composition. A monoclonal antibody composition displays a single binding specificity and affinity for a particular epitope.

As used herein, "isotype" refers to the antibody class (*e.g*. IgM, IgE, IgG such as IgG1 or IgG4) that is provided by the heavy chain constant region genes.

As used herein, an antibody or a protein that "specifically binds to an antigen", for example that "specifically binds to TfR" is intended to refer to an antibody or protein that binds to said antigen (for example human TfR of SEQ ID NO:16) with a KD of 100 nM or less, 10 nM or less, 1 nM or less.

The term "KD", as used herein, is intended to refer to the dissociation constant, which is obtained from the ratio of Kd to Ka (*i.e*. Kd/Ka) and is expressed as a molar concentration (M). KD values for antibodies can be determined using methods well established in the art. A method for determining the KD of an antibody is by using surface plasmon resonance, or using a biosensor system such as a Biacore^{®} system.

The term "Kassoc" or "Ka", as used herein, is intended to refer to the association rate of a particular antibody-antigen interaction, whereas the term "Kdis" or "Kd," as used herein, is intended to refer to the dissociation rate of a particular antibody-antigen interaction.

As used herein, the term "affinity" refers to the strength of interaction between antibody and antigen at single antigenic sites. Within each antigenic site, the variable region of the antibody "arm" interacts through weak non-covalent forces with the antigen at numerous sites; the more interactions, the stronger the affinity.

As used herein, the term "HL-60 cell line" refers to the promyelocytic derived by Collins et al. (PNAS 1978, 75:2458-1462) and also described in Gallagher et al. (Blood, 1979, 54:713-733), for example available at ATCC^{®} collection under catalog number CCL-240^{™}.

As used herein, the term "host cell" refers to prokaryotic or eukaryotic cells. Eukaryotic cells, for example mammalian host cells, yeast or filamentous fungi, are preferred, and in particular mammalian cells, because they are more likely than prokaryotic cells to assemble and secrete a properly folded and immunologically active antibody.

As used herein, the antibody "A24" refers to the antibody as disclosed in WO 2005/111082.

As used herein, the term "ADCC" or "antibody dependent cell cytotoxicity" activity refers to cell depleting activity. ADCC activity can be measured by ADCC assays commercially available, for example, ADCC Reporter Bioassay as commercialized by Promega under Ref# G7015.

As used herein, the term "apoptosis" refers to the programmed cell death. More information about apoptosis can be found in "Apoptosis: A Review of Programmed Cell Death, Susan Elmore, Toxicol Pathol. 2007; 35(4): 495-516.

As used herein, the term "EC₅₀" refers to the concentration of an antibody to induce a response, halfway between the baseline and maximum after a specified exposure time. For example, such concentration can be determined by GraphPad software.

As used herein, the term "subject" includes any human or non-human animal. The term "non-human animal" includes all vertebrates, e.g. mammals and non-mammals, such as non-human primates, sheep, dogs, cats, horses, cows, chickens, amphibians, reptiles, ... The term "subjects" also encompasses the term "patient" and is preferably a human.

As used herein, the term "bonded" or "bounded" refers to a linkage. This linkage is also represented by the dash "-" in formula (I) or (II) or (VIII). Linkage may be a covalent bond, or a non-covalent interaction such as through electrostatic forces. Preferably, bonds are covalent bonds. As used herein, the "wavy lines" on formulas represents the attachment sites between each part (e.g. Ab, L, Z, X and D) of the ADC of the disclosure.

As used herein, the term "linker" or "linker molecule" refers to any chemical moiety that is capable of covalently joining a compound, such as a drug moiety to another moiety such as an antibody moiety. Preferably, the linker is of formulas (III) or (V). Such linker molecules are appropriate to be linked to a compound comprising a thiol moiety, such as an antibody.

As used herein, the term "cleavable" refers to a linker which can be cleaved under specific environmental conditions (such as redox potential or pH) or specific lysosomal enzymes in response to intracellular environments, for example, after internalizing of the ADC in a cell.

As used herein, the term "non-cleavable" refers to a linker which will not be cleaved under specific environmental conditions. Such linkers are part of the payload.

As used herein, the term "dipeptide of valine-citrulline" (hereafter referred as "dipeptide VC") represents a linker consisting of two amino acids valine and citrulline. This dipeptide is notably of formula (VI):

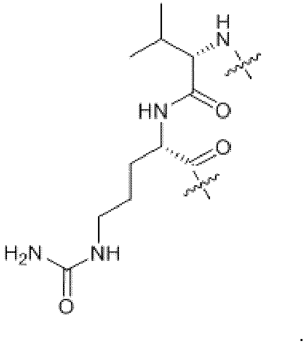

As used herein, the term "aminobenzyl ester self-immolative group" refers to an aminobenzyl ester group that functions as a self immolative group. Such group can be represented by a compound of formula (IV). A "self-immolative group" may be defined as a bifunctional chemical moiety which (i) is capable of covalently linking together at least two other chemical moieties into a stable molecule, (ii) can be released from one of the spaced chemical moieties from the molecule by means of enzymatic cleavage, and (iii) following enzymatic cleavage, can spontaneously cleave from the remainder of the molecule to release the other of the spaced chemical moieties.

As used herein, the term "alkyl" refers to a monovalent saturated hydrocarbon chain (having straight or branched chain). For example, alkyl refers to C₁-C₂₀ alkyl. Preferably, the alkyl is a "lower alkyl", i.e. an alkyl group having 1, 2, 3, 4, 5 or 6 carbons (straight or branched chain C₁-C₆ alkyl group). For example, this includes methyl, ethyl, n-propyl, i-propyl, n-butyl, i-butyl, sec-butyl, tert-butyl, n-pentyl, n-hexyl, and the like.

As used herein, the term "haloalkyl" refers to an alkyl having one or more hydrogen atoms replaced by one or halogen atoms. Preferably, the haloalkyl is a "lower haloalkyl", i.e. a haloalkyl group having 1, 2, 3, 4, 5 or 6 carbons (straight or branched chain C₁-C₆ haloalkyl group). For example, this includes CF₃, CF₂Br, CH₂F, CHFCH₃, CF₃CH₂, CF₃CF₂, CHF₂CF₂, CH₂Cl, or CH₂CH₂C1.

As used herein "therapeutically acceptable amount" refers to an amount sufficient to effect the desired results (for example a reduction in tumor size, inhibition of tumor growth, prevention of metastasis, induction of apoptosis, ...).

While the Drug-to-Antibody Ratio has in principle an exact value for a single ADC, it is understood that the value will often be an average value when used to describe a composition containing many ADCs, due to some degree of inhomogeneity, typically associated with the conjugation step. The average loading for a sample of an ADC is also referred to herein as the drug to antibody ratio, or "DAR". In some embodiments, the DAR is between about 1 and about 8 (*i.e.* 1, 1.5, 2, 2.5, 3, 3.5, 4, 4.5, 5, 5.5, 6, 6.5, 7, 7.5 and 8), preferably between about 4. Hence, in compositions comprising multiple copies of ADCs of Formula (I), "*p*" refers to the average number of -L'-D moieties per antibody Ab. For an average *p* number close to 4, the ADC will be considered to be a "DAR4". Methods for measuring the drug-antibody ratio are disclosed for example described in Conilh et al (Pharmaceuticals 2021, 14(3), 247).

Drug loading to the antibody via the linker L' may be limited by the number of attachments sites on the antibody moiety. In some embodiments, the linker moiety (L') of the ADC attaches to the antibody moiety through a chemically active group on one or more amino acid residues on the antibody moiety. For example, the linker may be attached to the antibody moiety via a free amino acid, imino, hydroxyl, thiol or carboxyl group (e.g. to the N- or C- terminus, to the epsilon amino group of one or more lysine residues, to the free carboxylic group of one or more of the glutamic acid or aspartic acid residues, or to the sulfhydryl group of one or more cysteine residues). The site to which the linker is attached can be natural residue in the amino acid sequence of the antibody moiety, or it can be introduced into the antibody moiety, e.g. by DNA recombinant technology (e.g. by introducing a cysteine or a non-natural amino acid residue into the amino acid sequence) or by protein biochemistry (e.g. by reduction, pH adjustment, or hydrolysis). When the attachment site is inter-chain cysteine thiol group, an antibody may have only one or few cysteine thiol groups through which a linker may be attached. Indeed, most reactive cysteine thiols generally exist as inter-chain disulfide bridges. Over-attachment of linker-toxin to the antibody may destabilize the antibody by reducing the cysteine residues available to form inter-chain disulfide bridge. Therefore, an optimal drug antibody ratio should increase potency of the ADC (by increasing the number of attached drug moieties per antibody) without destabilizing the antibody moiety and without degrading pharmacokinetics properties.

As used herein, the term "drug" also refers to "a payload", *i.e*. a moiety that is conjugated to an antibody (or a fragment). D is not to be construed as limited to classical chemical therapeutic agent. For example, D can encompass a protein, a peptide or a polypeptide possessing the desired biological activity. Preferably, it refers to a therapeutic moiety, such as a cytotoxin. A "cytotoxin" or "cytotoxic agent" includes any agent that is detrimental to *(e.g.* kills) cells.

Unless specified otherwise, the present disclosure encompasses the compounds, ADC, drug or cytotoxin as described herein and, their tautomers, enantiomers, diastereomers, racemates or mixtures thereof, and their hydrates, esters, solvates or pharmaceutically acceptable salts.

Any formula given herein is also intended to represent unlabeled as well as isotopically labeled forms of the compounds, like deuterium labeled compounds or ¹⁴C-labeled compounds.

The terms "pharmaceutically acceptable salts" refer to salts that retain the biological effectiveness and properties of the compounds of this disclosure and, which typically are not biologically or otherwise undesirable. In many cases, the compounds of the disclosure are capable of forming acid and/or base salts by virtue of the presence of amino and/or carboxyl groups or groups similar thereto. Pharmaceutically acceptable acid addition salts can be formed with organic acids and/or inorganic acids. Pharmaceutically acceptable base addition salts can be formed with organic bases and/or inorganic bases. Such salts are well-known from those skilled in the art.

### Recombinant antibodies

Antibodies of the disclosure are anti-TfR antibodies. Preferably, such antibodies include the humanized recombinant antibodies mAb1-mAb16, isolated and structurally characterized by their variable heavy and light chain amino acid sequences and human constant isotype as described in the **Table 1** below:

**Table 1: Variable heavy and light chain amino acid sequences of mAb1-mAb16**

| **Antibody** | **VH Amino acid sequence** | **VL Amino acid sequence** | **Isotype constant region** |
|---|---|---|---|
| mAb1 | SEQ ID NO:11 (VH4) | SEQ ID NO:13 (VL4) | IgG4 |
| mAb2 | SEQ ID NO:11 (VH4) | SEQ ID NO:14 (VL5) | IgG4 |
| mAb3 | SEQ ID NO:12 (VH5) | SEQ ID NO:13 (VL4) | IgG4 |
| mAb4 | SEQ ID NO:12 (VH5) | SEQ ID NO:15 (VL6) | IgG4 |
| mAb5 | SEQ ID NO:11 (VH4) | SEQ ID NO:13 (VL4) | IgG1 |
| mAb6 | SEQ ID NO:11 (VH4) | SEQ ID NO:14 (VL5) | IgG1 |
| mAb7 | SEQ ID NO:12 (VH5) | SEQ ID NO:13 (VL4) | IgG1 |
| mAb8 | SEQ ID NO:12 (VH5) | SEQ ID NO:15 (VL6) | IgG1 |
| mAb9 | SEQ ID NO:11 (VH4) | SEQ ID NO:13 (VL4) | IgG1 (AA) |
| mAb10 | SEQ ID NO:11 (VH4) | SEQ ID NO:14 (VL5) | IgG1 (AA) |
| mAb11 | SEQ ID NO:12 (VH5) | SEQ ID NO:13 (VL4) | IgG1 (AA) |
| mAb12 | SEQ ID NO:12 (VH5) | SEQ ID NO:15 (VL6) | IgG1 (AA) |
| mAb13 | SEQ ID NO:11 (VH4) | SEQ ID NO:13 (VL4) | IgG1 N297A |
| mAb14 | SEQ ID NO:11 (VH4) | SEQ ID NO:14 (VL5) | IgG1 N297A |
| mAb15 | SEQ ID NO:12 (VH5) | SEQ ID NO:13 (VL4) | IgG1 N297A |
| mAb16 | SEQ ID NO:12 (VH5) | SEQ ID NO:15 (VL6) | IgG1 N297A |

The corresponding amino acid and nucleotide coding sequence of the constant isotype regions of IgG4, IgG1 and their mutant versions IgG1 AA and IgG1 N297A used for generating mAb1 to mAb16 are well-known in the art. Full length light and heavy chains and corresponding coding sequences of mAb1 is shown in the **Table 2** below.

**Table 2: Full length heavy and light chain DNA coding sequences**

| **Antibody** | **Amino acid sequence** | **DNA coding sequence** |
|---|---|---|
| mAb1 | Heavy Chain: SEQ ID NO:18 | Heavy Chain: SEQ ID NO:20 |
| | Light Chain : SEQ ID NO:17 | Light Chain : SEQ ID NO:19 |

Examples of the amino acid sequences of the VH CDR1s (also called HCDR1), VH CDR2s (also called HCDR2), VH CDR3s (also called HCDR1), VL CDR1s (also called LCDR1), VL CDR2s (also called LCDR2), VL CDR3s (also called HCDR3) of some antibodies according to the disclosure are shown in **Table 3.**

In **Table 3,** the CDR regions of some antibodies of the present disclosure are delineated using the Chothia system (Chothia C, Lesk AM. 1987, J Mol Biol 196, 901-917). For the ease of reading, the CDR regions are called hereafter HCDR1, HCDR2, HCDR3, LCDR1, LCDR2, LCDR3 respectively.

**Table 3: CDR regions of mAb1 to mAb16 and reference A24 antibody according to Chothia**

| **Original antibody** | **HCDR1** | **HCDR2** | **HCDR3** | **LCDR1** | **LCDR2** | **LCDR3** |
|---|---|---|---|---|---|---|
| A24 | SEQ ID NO:1 | SEQ ID NO:2 | SEQ ID NO:3 | SEQ ID NO:4 | SEQ ID NO:7 | SEQ ID NO:6 |
| mAb1 | SEQ ID NO:1 | SEQ ID NO:2 | SEQ ID NO:3 | SEQ ID NO:4 | SEQ ID NO:5 | SEQ ID NO:6 |
| mAb5 | | | | | | |
| mAb9 | | | | | | |
| mAb13 | | | | | | |
| mAb2 | SEQ ID NO:1 | SEQ ID NO:2 | SEQ ID NO:3 | SEQ ID NO:4 | SEQ ID NO:8 | SEQ ID NO:6 |
| mAb6 | | | | | | |
| mAb10 | | | | | | |
| mAb14 | | | | | | |
| mAb3 | SEQ ID NO:1 | SEQ ID NO:2 | SEQ ID NO:3 | SEQ ID NO:4 | SEQ ID NO:5 | SEQ ID NO:6 |
| mAb7 | | | | | | |
| mAb11 | | | | | | |
| mAb15 | | | | | | |
| mAb4 | SEQ ID NO:1 | SEQ ID NO:2 | SEQ ID NO:3 | SEQ ID NO:4 | SEQ ID NO:7 | SEQ ID NO:6 |
| mAb8 | | | | | | |
| mAb12 | | | | | | |
| mAb16 | | | | | | |

**Tables 4, 5** and **6** describe useful amino acid and nucleotides sequences relative to antibodies of ADC.

**Table 4: Brief description of useful amino acid and nucleotide sequences for practicing the invention**

| SEQ ID NO: | Description of the sequence |
|---|---|
| 1 | HCDR1 amino acid sequence of A24, VH4 and VH5 |
| 2 | HCDR2 amino acid sequence of A24, VH4 and VH5 |
| 3 | HCDR3 amino acid sequence of A24, VH4 and VH5 |
| 4 | LCDR1 amino acid sequence of A24, VL4, VL5 and VL6 |
| 5 | LCDR2 amino acid sequence of VL4 |
| 6 | LCDR3 amino acid sequence of A24, VL4, VL5 and VL6 |
| 7 | LCDR2 amino acid sequence of A24 |
| 8 | LCDR2 amino acid sequence of VL5 |
| 9 | VH0 amino acid sequence of A24 |
| 10 | VL0 amino acid sequence of A24 |
| 11 | VH4 amino acid sequence |
| 12 | VH5 amino acid sequence |
| 13 | VL4 amino acid sequence |
| 14 | VL5 amino acid sequence |
| 15 | VL6 amino acid sequence |
| 16 | Human transferrin receptor amino acid sequence |
| 17 | Full length light chain of mAb1, mAb3, mAb5, mAb7, mAb9, mAb11, mAb13, mAb15 (with VL4) |
| 18 | Full length heavy chain of mAb1, mAb2 (with VH4-IgG4 isotype) |
| 19 | Nucleotide sequence encoding Full length light chain of mAb1, mAb3, mAb5, mAb7, mAb9, mAb11, mAb13, mAb15 (with VL4) of SEQ ID NO:19 |
| 20 | Nucleotide sequence encoding Full length heavy chain of mAb1, mAb2 (with VH4-IgG4 isotype) of SEQ ID NO:22 |

**Table 5: Brief description of useful amino acid and nucleotide sequences for practicing the invention**

| SEQ ID NO: | **Describes the amino acid or nucleotide sequence below:** |
|---|---|
| 1 | GYTFTNQ |
| 2 | NTYTGE |
| 3 | EGWDSMDY |
| 4 | SASSSVNYMH |
| 5 | STSNRAT |
| 6 | QQRSSYPLT |
| 7 | STSNLAS |
| 8 | STSNRAS |
| 9 | QIQLVQSGPELKKPGETVKISCKASGYTFTNQGMNWVKQAPGKGLKWMGWINTYTGE PINADDFKGRFAISLETSASTAYLQINNLKNEDMATYFCVREGWDSMDYWGQGTSVTV SS |
| 10 | QIVLTQSPAIMSASPGEKVTITCSASSSVNYMHWFQQKPGTSPKLWIYSTSNLASGVPA RFSGSGSGTSYSLTISRMEAEDAATYYCQQRSSYPLTFGAGTKLELKR |
| 11 | QVQLVQSGPELKKPGASVKVSCKASGYTFTNQGMNWVKQAPGKGLKWMGWINTYTG EPINADDFKGRFVISLDTSASTAYLQISSLKAEDTAVYFCVREGWDSMDYWGQGTSVTV SS |
| 12 | MEWSWVFLFFLSVTTGVHSQVQLVQSGPELKKPGASVKVSCKASGYTFTNQGMNWV KQAPGKGLKWMGWINTYTGEPINADDFKGRFVISLETSASTAYLQISNLKNEDTAVYFC VREGWDSMDYWGQGTSVTVSS |
| 13 | QIVLTQSPATLSVSPGERATLSCSASSSVNYMHWFQQKPGQSPRLLIYSTSNRATGIPA RFSGSGSGTSYTLTISSLEPEDFAVYYCQQRSSYPLTFGQGTKLEIKR |
| 14 | QIVLTQSPATLSLSPGERATLSCSASSSVNYMHWFQQKPGQSPRLLIYSTSNRASGVPA RFSGSGSGTSYTLTISRLEPEDFAVYYCQQRSSYPLTFGQGTKLEIKR |
| 15 | QIVLTQSPATLSLSPGERATLSCSASSSVNYMHWFQQKPGQSPRLLIYSTSNLASGVPA RFSGSGSGTSYTLTISRLEPEDAAVYYCQQRSSYPLTFGAGTKLELKR |
| 16 | MMDQARSAFSNLFGGEPLSYTRFSLARQVDGDNSHVEMKLAVDEEENADNNTKANVT KPKRCSGSICYGTIAVIVFFLIGFMIGYLGYCKGVEPKTECERLAGTESPVREEPGEDFP AARRLYWDDLKRKLSEKLDSTDFTGTIKLLNENSYVPREAGSQKDENLALYVENQFREF KLSKVWRDQHFVKIQVKDSAQNSVIIVDKNGRLVYLVENPGGYVAYSKAATVTGKLVHA NFGTKKDFEDLYTPVNGSIVIVRAGKITFAEKVANAESLNAIGVLIYMDQTKFPIVNAELSF FGHAHLGTGDPYTPGFPSFNHTQFPPSRSSGLPNIPVQTISRAAAEKLFGNMEGDCPS DWKTDSTCRMVTSESKNVKLTVSNVLKEIKILNIFGVIKGFVEPDHYVVVGAQRDAWGP GAAKSGVGTALLLKLAQMFSDMVLKDGFQPSRSIIFASWSAGDFGSVGATEWLEGYLS SLHLKAFTYINLDKAVLGTSNFKVSASPLLYTLIEKTMQNVKHPVTGQFLYQDSNWASKV EKLTLDNAAFPFLAYSGIPAVSFCFCEDTDYPYLGTTMDTYKELIERIPELNKVARAAAEV AGQFVIKLTHDVELNLDYERYNSQLLSFVRDLNQYRADIKEMGLSLQWLYSARGDFFRA TSRLTTDFGNAEKTDRFVMKKLNDRVMRVEYHFLSPYVSPKESPFRHVFWGSGSHTLP ALLENLKLRKQNNGAFNETLFRNQLALATWTIQGAANALSGDVWDIDNEF |
| 17 | MSVPTQVLGLLLLWLTDARCQIVLTQSPATLSVSPGERATLSCSASSSVNYMHWFQQK PGQSPRLLIYSTSNRATGIPARFSGSGSGTSYTLTISSLEPEDFAVYYCQQRSSYPLTFG QGTKLEIKRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNRGEC |
| 18 | MEWSWVFLFFLSVTTGVHSQVQLVQSGPELKKPGASVKVSCKASGYTFTNQGMNWV KQAPGKGLKWMGWINTYTGEPINADDFKGRFVISLDTSASTAYLQISSLKAEDTAVYFC VREGWDSMDYWGQGTSVTVSSASTKGPSVFPLAPCSRSTSESTAALGCLVKDYFPEP VTVSWNSGALTSGVHTFPAVLQSSGLYSLSSVVTVPSSSLGTKTYTCNVDHKPSNTKV DKRVESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVSNKGLPS SIEKTISKAKGQPREPQVYTLPPSQEEMTKNQVSLTCLVKGFYPSDIAVEWESNGQPEN NYKTTPPVLDSDGSFFLYSRLTVDKSRWQEGNVFSCSVMHEALHNHYTQKSLSLSLG |
| 19 | AAGCTTGCCGCCACCATGTCCGTGCCTACCCAGGTGCTGGGACTGCTGCTGCTGT GGCTGACCGATGCCAGGTGCCAGATCGTGCTGACCCAGTCTCCTGCCACCCTGTC TGTGTCTCCCGGCGAGAGAGCTACCCTGTCCTGCTCCGCCTCCTCCTCCGTGAACT ACATGCACTGGTTCCAGCAGAAGCCCGGCCAGTCCCCCAGACTGCTGATCTACTCC ACCTCCAACCGGGCCACCGGCATCCCTGCCAGATTTTCCGGCTCTGGCTCCGGCA CCTCCTATACCCTGACCATCTCCAGCCTGGAACCCGAGGACTTCGCCGTGTACTAC TGCCAGCAGCGGTCCTCCTACCCCCTGACCTTTGGCCAGGGCACCAAGCTGGAAA TCAAGCGTACGGTGGCCGCTCCCAGCGTGTTCATCTTCCCCCCAAGCGACGAGCA GCTGAAGAGCGGCACCGCCAGCGTGGTGTGTCTGCTGAACAACTTCTACCCCAGG GAGGCCAAGGTGCAGTGGAAGGTGGACAACGCCCTGCAGAGCGGCAACAGCCAG GAGAGCGTCACCGAGCAGGACAGCAAGGACTCCACCTACAGCCTGAGCAGCACCC TGACCCTGAGCAAGGCCGACTACGAGAAGCACAAGGTGTACGCCTGTGAGGTGAC |
| | CCACCAGGGCCTGTCCAGCCCCGTGACCAAGAGCTTCAACAGGGGCGAGTGCTGA TGAATTC |
| 20 | AAGCTTGCCGCCACCATGGAATGGTCCTGGGTGTTCCTGTTCTTCCTGTCCGTGAC CACCGGCGTGCACTCCCAGGTGCAGCTGGTGCAGTCTGGCCCCGAGCTGAAGAAA CCTGGCGCCTCCGTGAAGGTGTCCTGCAAGGCTTCCGGCTACACCTTTACAAACCA GGGCATGAACTGGGTCAAGCAGGCCCCTGGCAAGGGCCTGAAGTGGATGGGCTG GATCAACACCTACACCGGCGAGCCCATCAACGCCGACGACTTCAAGGGCAGATTC GTGATCTCCCTGGACACCTCCGCCTCCACCGCCTACCTGCAGATCAGCTCTCTGAA GGCCGAGGATACCGCCGTGTACTTCTGCGTGCGGGAAGGCTGGGACTCCATGGAC TATTGGGGCCAGGGCACCTCCGTGACCGTGTCTAGCGCTTCTACAAAGGGCCCAA GCGTGTTCCCCCTGGCCCCCTGCTCCAGAAGCACCAGCGAGAGCACAGCCGCCCT GGGCTGCCTGGTGAAGGACTACTTCCCCGAGCCCGTGACCGTGTCCTGGAACAGC GGAGCCCTGACCAGCGGCGTGCACACCTTCCCCGCCGTGCTGCAGAGCAGCGGC CTGTACAGCCTGAGCAGCGTGGTGACCGTGCCCAGCAGCAGCCTGGGCACCAAGA CCTACACCTGTAACGTGGACCACAAGCCCAGCAACACCAAGGTGGACAAGAGGGT GGAGAGCAAGTACGGCCCACCCTGCCCCCCCTGCCCAGCCCCCGAGTTCCTGGGC GGACCCAGCGTGTTCCTGTTCCCCCCCAAGCCCAAGGACACCCTGATGATCAGCA GAACCCCCGAGGTGACCTGTGTGGTGGTGGACGTGTCCCAGGAGGACCCCGAGGT CCAGTTCAACTGGTACGTGGACGGCGTGGAGGTGCACAACGCCAAGACCAAGCCC AGAGAGGAGCAGTTTAACAGCACCTACCGGGTGGTGTCCGTGCTGACCGTGCTGC ACCAGGACTGGCTGAACGGCAAAGAGTACAAGTGTAAGGTCTCCAACAAGGGCCT GCCAAGCAGCATCGAAAAGACCATCAGCAAGGCCAAGGGCCAGCCTAGAGAGCCC CAGGTCTACACCCTGCCACCCAGCCAAGAGGAGATGACCAAGAACCAGGTGTCCC TGACCTGTCTGGTGAAGGGCTTCTACCCAAGCGACATCGCCGTGGAGTGGGAGAG CAACGGCCAGCCCGAGAACAACTACAAGACCACCCCCCCAGTGCTGGACAGCGAC GGCAGCTTCTTCCTGTACAGCAGGCTGACCGTGGACAAGTCCAGATGGCAGGAGG GCAACGTCTTTAGCTGCTCCGTGATGCACGAGGCCCTGCACAACCACTACACCCAG AAGAGCCTGAGCCTGTCCCTGGGCTGATGAATTC |

**Table 6: Description of the variable regions and IgG Fc region of mAb1-mAb16**

| **Example** | **Variable region and IgG Fc region** |
|---|---|
| mAb1 | VH4/VL4 with IgG4 Fc region |
| mAb2 | VH4/VL5 with IgG4 Fc region |
| mAb3 | VH5/VL4 with IgG4 Fc region |
| mAb4 | VH5/VL6 with IgG4 Fc region |
| mAb5 | VH4/VL4 with IgG1 Fc region |
| mAb6 | VH4/VL5 with IgG1 Fc region |
| mAb7 | VH5/VL4 with IgG1 Fc region |
| mAb8 | VH5/VL6 with IgG1 Fc region |
| mAb9 | VH4/VL4 with IgG1 AlaAla mutant Fc region |
| mAb10 | VH4/VL5 with IgG1 AlaAla mutant Fc region |
| mAb11 | VH5/VL4 with IgG1 AlaAla mutant Fc region |
| mAb12 | VH5/VL6 with IgG1 AlaAla mutant Fc region |
| mAb13 | VH4/VL4 with IgG1 N297A mutant Fc region |
| mAb14 | VH4/VL5 with IgG1 N297A mutant Fc region |
| mAb15 | VH5/VL4 with IgG1 N297A mutant Fc region |
| mAb16 | VH5/VL6 with IgG1 N297A mutant Fc region |

In one embodiment, an isolated recombinant antibody has: a heavy chain variable region comprising HCDR1 of SEQ ID NO: 1; HCDR2 of SEQ ID NO: 2; HCDR3 of SEQ ID NO: 3; a light chain variable region comprising LCDR1 of SEQ ID NO: 4; LCDR2 of SEQ ID NOs: 5 or 8; and LCDR3 of SEQ ID NOs: 6; wherein said antibody specifically binds to the transferrin receptor of SEQ ID NO:16.

In specific embodiments, the isolated recombinant antibody according to the disclosure comprises either:
(a) a variable heavy chain polypeptide comprising HCDR1 of SEQ ID NO:1, HCDR2 of SEQ ID NO:2, HCDR3 of SEQ ID NO:3 and a variable light chain polypeptide comprising LCDR1 of SEQ ID NO:4, LCDR2 of SEQ ID NO:5 and LCDR3 of SEQ ID NO:6;
(b) a variable heavy chain polypeptide comprising HCDR1 of SEQ ID NO:1, HCDR2 of SEQ ID NO:2, HCDR3 of SEQ ID NO:3 and a variable light chain polypeptide comprising LCDR1 of SEQ ID NO:4, LCDR2 of SEQ ID NO:8 and LCDR3 of SEQ ID NO:6;
(c) a variable heavy chain polypeptide comprising VH of SEQ ID NO:11 and a variable light chain polypeptide VL of SEQ ID NO:13;
(d) a variable heavy chain polypeptide comprising VH of SEQ ID NO:11 and a variable light chain polypeptide VL of SEQ ID NO:14;
(e) a variable heavy chain polypeptide comprising VH of SEQ ID NO:11 and a variable light chain polypeptide VL of SEQ ID NO:15;
(f) a variable heavy chain polypeptide comprising VH of SEQ ID NO:12 and a variable light chain polypeptide VL of SEQ ID NO:13;
(g) a variable heavy chain polypeptide comprising VH of SEQ ID NO:12 and a variable light chain polypeptide VL of SEQ ID NO:14; or
(h) a variable heavy chain polypeptide comprising VH of SEQ ID NO:12 and a variable light chain polypeptide VL of SEQ ID NO:15.

In a specific embodiment, said recombinant anti-TfR antibody as defined above have one or more of the following properties:
(i) it binds to the transferrin receptor with a KD of 10nM or less, preferably with a KD of 1nM or less, as measured by SPR;
(ii) it binds to the transferrin receptor with an EC₅₀ of 0.1 µg/ml or below, preferably of 0.05 µg/ml or below, as measured in an ELISA assay (see PCT/EP2016/067465 for more information);
(iii) it induces apoptosis of HL-60 cell line to a level equal or superior to the induction level measured with the corresponding reference chimeric antibody having the parental murine variable regions with VH of SEQ ID NO:9 and VL of SEQ ID NO:10, for example as measured using the HL-60 apoptosis induction assay. Typically, an amount of 10 µg/ml of a recombinant antibody of the present disclosure may be assayed for induction of apoptosis of HL-60 cell line as compared to the same amount of the reference chimeric antibody with the parental murine variable regions of A24 comprising VH of SEQ ID NO:9 and VL of SEQ ID NO:10. Induction of apoptosis in the HL-60 apoptosis induction assay of a tested antibody is equal to a reference antibody if the percentage of positive cells as measured with the tested antibody is not significantly lower that the percentage of positive cells as measured with the reference antibody.

As used herein, a "corresponding" reference chimeric antibody refers to the reference antibody with an isotype constant region 100% identical to the isotype constant region of the antibody to be tested for a particular property, for example induction of apoptosis.

In certain embodiments that may be combined with the previous embodiments, an antibody provided herein is an antibody fragment of the above-defined antibodies. Antibody fragments include, but are not limited to, Fab, Fab', Fab'-SH, F(ab')2, Fv, UniBody, and scFv fragments, diabodies, single domain or nanobodies and other fragments. The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see, e.g., U.S. Patent No. 6,248,516 B1). Antibody fragments can be made by various techniques, including but not limited to proteolytic digestion of an intact antibody as well as production by recombinant host cells as described herein.

In certain embodiments, the antibody of the present disclosure is a humanized antibody. Typically, a non-human antibody is humanized to reduce immunogenicity to humans, while having at least the same affinity (or superior affinity) of the parental non-human antibody. In preferred embodiments, the antibodies of the present disclosure are humanized antibodies of the parent antibody A24. Generally, a humanized antibody comprises one or more variable domains in which, CDRs, (or portions thereof) are derived from a non-human antibody, *e.g.* the murine A24 antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g. the A24 antibody from which the CDR residues are derived), e.g., to restore or improve antibody specificity or affinity. In some specific embodiments, some CDR residues in a humanized antibody are also substituted, *e.g.* to restore or improve antibody specificity or affinity. Humanized antibodies and methods of making them are reviewed, *e.g.* in Almagro and Fransson, Front. Biosci. 13: 1619-1633 (2008), and are further described, e.g., in Riechmann et al., Nature 332:323-329 (1988); Queen et al., Proc. Natl Acad. Sci. USA 86: 10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling). Preferably the recombinant antibody according to the disclosure is a humanized silent antibody, preferably a humanized silent IgG1 or IgG4 antibody.

As used herein, the term "silent" antibody refers to an antibody that exhibits no or low ADCC activity as measured in an ADCC activity assay.

In one embodiment, the term "no or low ADCC activity" means that the silent antibody exhibits an ADCC activity that is at least below 10%, for example below 50% of the ADCC activity that is observed with the corresponding antibody with wild type human IgG1 isotype.

Silenced effector functions can be obtained by mutation in the Fc constant part of the antibodies and have been described in the Art: Strohl 2009 (AA & N297A); Baudino 2008, D265A (Baudino et al., J.Immunol. 181 (2008): 6664-69, Strohl, CO Biotechnology 20 (2009): 685-91). Examples of silent IgG1 antibodies comprise the so-called AA mutant comprising L234A and L235A mutation in the IgG1 Fc amino acid sequence. Another silent IgG1 antibody comprises the N297A mutation, which results in aglycosylated or non-glycosylated antibodies.

Antibodies with mutant amino acid sequences can be obtained by mutagenesis (e.g. site-directed or PCR-mediated mutagenesis) of the coding nucleic acid molecules, followed by testing of the encoded altered antibody for retained function (i.e. the functions set forth above) using the functional assays described herein.

### Antibodies with conservative modifications

In certain embodiments, an antibody (or a binding protein comprising antigen binding portion thereof) of the disclosure has a heavy chain variable region comprising HCDR1, HCDR2, and HCDR3 sequences and a light chain variable region comprising LCDR1, LCDR2, and LCDR3 sequences, wherein one or more of these CDR sequences have specified amino acid sequences based on the mAb1 to mAb16 antibodies described herein or conservative modifications thereof, and wherein the antibody or protein retains the desired functional properties of the anti-TfR antibodies of the disclosure.

Desired functional properties of the anti-TfR antibodies includes without limitation:
(i) it binds to the transferrin receptor with a KD of 10nM or less, preferably with a K_{D} of 1nM or less, for example as measured by SPR assay, for example using Biacore^{®};
(ii) it binds to the transferrin receptor with an EC₅₀ of 0.1 µg/ml or below, preferably of 0.05 µg/ml or below, as measured in an ELISA assay (see PCT/EP2016/067465 for more information);
(iii) it induces apoptosis of HL-60 cell line to a level equal or superior to the induction level measured with the corresponding reference chimeric antibody having the parental murine variable regions with VH of SEQ ID NO:9 and VL of SEQ ID NO:10, for example as measured using the HL-60 apoptosis induction assay. Typically, an amount of 10µg/ml of a recombinant antibody of the present disclosure may be assayed for induction of apoptosis of HL-60 cell line as compared to the same amount of the reference chimeric antibody with the parental murine variable regions of A24 comprising VH of SEQ ID NO:9 and VL of SEQ ID NO:10. Induction of apoptosis in the HL-60 apoptosis induction assay of a tested antibody is equal to a reference antibody if the percentage of positive cells as measured with the tested antibody is not significantly lower that the percentage of positive cells as measured with the reference antibody.

As used herein, the term "conservative sequence modifications" is intended to refer to amino acid substitutions in which the amino acid residue is replaced with an amino acid residue having a similar side chain. Families of amino acid residues having similar side chains have been defined in the art. These families include amino acids with basic side chains (*e.g*. lysine, arginine, histidine), acidic side chains (*e.g*. aspartic acid, glutamic acid), uncharged polar side chains (*e.g*. glycine, asparagine, glutamine, serine, threonine, tyrosine, cysteine, tryptophan), nonpolar side chains (*e.g*. alanine, valine, leucine, isoleucine, proline, phenylalanine, methionine), beta-branched side chains (*e.g*. threonine, valine, isoleucine) and aromatic side chains (*e.g*. tyrosine, phenylalanine, tryptophan, histidine). Thus, one or more amino acid residues within the CDR regions of an antibody of the disclosure can be replaced with other amino acid residues from the same side chain family, and the altered antibody can be tested for retained function using the functional assays described herein.

Modifications can be introduced into an antibody of the disclosure by standard techniques known in the art, such as site-directed mutagenesis and PCR-mediated mutagenesis.

### Framework or Fc engineering

Engineered antibodies of the disclosure include those in which modifications have been made to framework residues within VH and/or VL, *e.g.* to improve the properties of the antibody. Typically such framework modifications are made to decrease the immunogenicity of the antibody. For example, one approach is to "backmutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived. To return the framework region sequences to their germline configuration, the somatic mutations can be "backmutated" to the germline sequence by, for example, site-directed mutagenesis or PCR-mediated mutagenesis. Such "backmutated" antibodies are also intended to be encompassed by the invention.

Another type of framework modification involves mutating one or more residues within the framework region, or even within one or more CDR regions, to remove T cell-epitopes to thereby reduce the potential immunogenicity of the antibody.

In particular, the company Antitope (Cambridge UK) has developed a range of proprietary technologies for assessing and removing immunogenicity, which are based on identifying the location of T cell epitopes in therapeutic antibodies and proteins. These technologies are summarized below:
- iTope^{™} - an in silico technology for prediction of peptide binding to human MHC class II alleles (Perry et al. 2008 Drugs in R&D, 9(6):385-396).
- TCED^{™} - a database of known T cell epitopes identified in studies using EpiScreen^{™} T cell epitope mapping assays especially of antibody V regions (Bryson et al. 2010 Biodrugs 24(1):1-8). The database can be interrogated by BLAST searching to identify common motifs (Altschul et al. 1997 Nucleic Acids Res. (1997) 25:3389-3402).

In addition or alternative to modifications made within the framework or CDR regions, antibodies of the disclosure may be engineered to include modifications within the Fc region, typically to alter one or more functional properties of the antibody, such as serum half-life, complement fixation, Fc receptor binding, and/or antigen-dependent cellular cytotoxicity.

Furthermore, an antibody of the disclosure may be chemically modified (*e.g*. one or more chemical moieties can be attached to the antibody) or be modified to alter its glycosylation, again to alter one or more functional properties of the antibody. Each of these embodiments is described in further detail below.

As used herein, the term "isotype constant region" or "Fc region" is used interchangeably to define the C-terminal region of an immunoglobulin heavy chain, including native sequence Fc region and variant Fc regions. The human IgG heavy chain Fc region is generally defined as comprising the amino acid residue from position C226 or from P230 to the carboxyl-terminus of the IgG antibody. The numbering of residues in the Fc region is that of the EU index of Kabat. The C-terminal lysine (residue K447) of the Fc region may be removed, for example, during production or purification of the antibody. Accordingly, a composition of antibodies of the disclosure may comprise antibody populations with all K447 residues removed, antibody populations with no K447 residues removed, and antibody populations having a mixture of antibodies with and without the K447 residue.

In one specific embodiment, the hinge region of CH1 is modified such that the number of cysteine residues in the hinge region is altered, *e.g*. increased or decreased. This approach is described further in U.S. Patent No. 5,677,425 by Bodmer et al. The number of cysteine residues in the hinge region of CH1 is altered to, for example, facilitate assembly of the light and heavy chains or to increase or decrease the stability of the antibody.

In another embodiment, the Fc hinge region of an antibody is mutated to decrease the biological half-life of the antibody. More specifically, one or more amino acid mutations are introduced into the CH2-CH3 domain interface region of the Fc-hinge fragment such that the antibody has impaired Staphylococcyl protein A (SpA) binding relative to native Fc-hinge domain SpA binding. This approach is described in further detail in U.S. Patent No. 6,165,745 by Ward et al.

In another embodiment, the antibody is modified to increase its biological half-life. Various approaches are possible. For example, one or more of the following mutations can be introduced: T252L, T254S, T256F, as described in U.S. Patent No. 6,277,375 to Ward. Alternatively, to increase the biological half-life, the antibody can be altered within the CH1 or CL region to contain a salvage receptor binding epitope taken from two loops of a CH2 domain of an Fc region of an IgG, as described in U.S. Patent Nos. 5,869,046 and 6,121,022 by Presta et al.

In yet other embodiments, the Fc region is altered by replacing at least one amino acid residue with a different amino acid residue to alter the effector functions of the antibody. For example, one or more amino acids can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in further detail in U.S. Patent Nos. 5,624,821 and 5,648,260, both by Winter et al.

In another embodiment, one or more amino acids selected from amino acid residues can be replaced with a different amino acid residue such that the antibody has altered C1q binding and/or reduced or abolished complement dependent cytotoxicity (CDC). This approach is described in further detail in U.S. Patent Nos. 6,194,551 by Idusogie et al.

In another embodiment, one or more amino acid residues are altered to thereby alter the ability of the antibody to fix complement. This approach is described further in PCT Publication WO 94/29351 by Bodmer et al.

In yet another embodiment, the Fc region is modified to increase the ability of the antibody to mediate antibody dependent cellular cytotoxicity (ADCC) and/or to increase the affinity of the antibody for an Fcγ receptor by modifying one or more amino acids. This approach is described further in PCT Publication WO 00/42072 by Presta. Moreover, the binding sites on human IgG1 for FcγRI, FcγRII, FcγRIII and FcRn have been mapped and variants with improved binding have been described (see Shields, R.L. et al., 2001 J. Biol. Chem 276:6591-6604).

In other embodiments, the Fc region is modified to decrease the ability of the antibody to mediate antibody dependent cellular cytotoxicity (ADCC) and/or to decrease the affinity of the antibody for an Fcγ receptor by modifying one or more amino acids. Such antibodies with decreased effector functions, and in particular decreased ADCC include silent antibodies.

In certain embodiments, the Fc domain of the IgG1 isotype is used. In some specific embodiments, a mutant variant of the IgG1 Fc fragment is used, *e.g.* a silent IgG1 Fc which reduces or eliminates the ability of the fusion polypeptide to mediate antibody dependent cellular cytotoxicity (ADCC) and/or to bind to an Fcγ receptor. An example of an IgG1 isotype silent mutant is IgG1 wherein Leucine is replaced by Alanine at amino acid positions 234 and 235 as described in J. Virol 2001 Dec;75(24):12161-8 by Hezareh *et al.*

In certain embodiments, the Fc domain is a silent Fc mutant preventing glycosylation at position 297 of the Fc domain. For example, the Fc domain contains an amino acid substitution of asparagine at position 297. An example of such amino acid substitution is the replacement of N297 by a glycine or an alanine.

In still another embodiment, the glycosylation of an antibody is modified. For example, an aglycoslated antibody can be made (*i.e*. the antibody lacks glycosylation). Glycosylation can be altered to, for example, increase the affinity of the antibody for the antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. Such an approach is described in further detail in U.S. Patent Nos. 5,714,350 and 6,350,861 by Co et al.

Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the disclosure to thereby produce an antibody with altered glycosylation. For example, EP 1 176 195 by Hang et al. describes a cell line with a functionally disrupted FUT8 gene, which encodes a fucosyl transferase, such that antibodies expressed in such a cell line exhibit hypofucosylation. Therefore, in one embodiment, the antibodies of the disclosure are produced by recombinant expression in a cell line which exhibits a hypofucosylation pattern, for example, a mammalian cell line with deficient expression of the FUT8 gene encoding fucosyltransferase. PCT Publication WO 03/035835 by Presta describes a variant CHO cell line, Lecl3 cells, with reduced ability to attach fucose to Asn(297)-linked carbohydrates, also resulting in hypofucosylation of antibodies expressed in that host cell (see also Shields, R.L. et al., 2002 J. Biol. Chem. 277:26733-26740). PCT Publication WO 99/54342 by Umana et al. describes cell lines engineered to express glycoprotein-modifying glycosyl transferases (*e.g*. beta(1,4)-N acetylglucosaminyltransferase III (GnTIII)) such that antibodies expressed in the engineered cell lines exhibit increased bisecting GlcNac structures which results in increased ADCC activity of the antibodies (see also Umana et al., 1999 Nat. Biotech. 17:176-180).

Another modification of the antibodies herein that is contemplated by the present disclosure is pegylation or hesylation or related technologies. An antibody can be pegylated to, for example, increase the biological (e.g., serum) half-life of the antibody. To pegylate an antibody, the antibody, or fragment thereof, typically is reacted with polyethylene glycol (PEG), such as a reactive ester or aldehyde derivative of PEG, under conditions in which one or more PEG groups become attached to the antibody or antibody fragment. The pegylation can be carried out by an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C₁-C₁₀) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies of the disclosure. See for example, EP 0 154 316 by Nishimura et al. and EP 0 401 384 by Ishikawa et al.

Another modification of the antibodies that is contemplated by the present disclosure is a conjugate or a protein fusion of at least the antigen-binding region of the antibody of the disclosure to serum protein, such as human serum albumin or a fragment thereof to increase half-life of the resulting molecule. Such approach is for example described in Ballance et al. EP 0 322 094.

Another possibility is a fusion of at least the antigen-binding region of the antibody of the disclosure to proteins capable of binding to serum proteins, such human serum albumin to increase half-life of the resulting molecule. Such approach is for example described in Nygren et al., EP 0 486 525.

In one specific embodiment, the effector function or complement activation function of an antibody according to the disclosure has been reduced or eliminated relative to a wild-type antibody of the same isotype. In one aspect, the effector function is reduced or eliminated by a method selected from reduction of glycosylation of the antibody, modification of the antibody isotype to an isotype that naturally has reduced or eliminated effector function, and modification of the Fc region. In specific related embodiment, said isotype with reduced or eliminated effector function is IgG4 isotype.

### Linkers

In an embodiment of the disclosure, ADC comprises a linker L' which is preferably a cleavable linker. In an embodiment of the disclosure, such linker L' corresponds to the formula (VIII): L-Z-X. In an embodiment of the disclosure, ADC contains three different linkers: L, Z and X. in a preferred embodiment, the general formula of these three linkers linked together is represented by formula (IX), reproduced below:

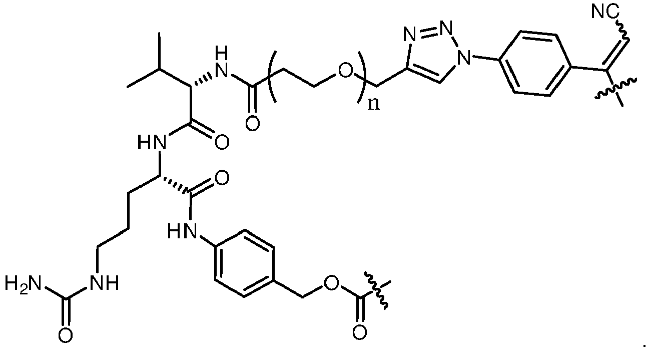

Such linkers allow linkage between the antibody and the drug. Preferably, the linkage is covalent. Therefore, in an embodiment of the invention, D is covalently bonded to X and/or X is covalently bonded to Z and/or Z is covalently bonded to L and/or L is covalently bonded to Ab. More preferably, D is covalently bonded to X and X is covalently bonded to Z and Z is covalently bonded to L and L is covalently bonded to Ab.

L is a linker molecule of formula (III) or (V), wherein n is an integer comprised between 2 and 20. L is a PEG-arylpropiolonitril, also called "APN". As disclosed herein, n encompasses then 2, 3, 4, 5, 6, 7, 8, 9, 10, 11, 12, 13, 14, 15, 16, 17, 18, 19 and 20. L confers stability of ADC in a wide range of conditions. More information about L is disclosed for example in PCT/EP2014/064387.

In an embodiment, which can be combined with other embodiments, L is linked to antibody *via* at least one thiol moiety (from an antibody's cystein). In an embodiment, linkage between L and the antibody is on the side of the double bond C=C in formula (III) or (V).

Z is a dipeptide of valine-citrulline bonded to L. Z is linked to L on the side of carbonyl group (i.e. carbonyl function) in L. In an embodiment, linkage between L and Z occurs between the carbonyl group of L and the amino function of Z (*e.g.* amino function of valine). Dipeptide VC is a cleavable linker. Cleavable linkers exploit the differences between conditions in the blood stream and the cytoplasmic conditions within target cells (notably cancer cells). Dipeptide VC is cleaved in the acidic environment within lysosomes by lysosomal proteases, such as cathepsin B. After internalization of ADC in target cells, there is an intracellular cleavage mechanism by cathepsin B, between Z and X (*e.g.* cleavage between the citrulline and aminobenzyl ester self-immolative group on the side of the amine function (see 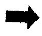 in formula (VII) below:

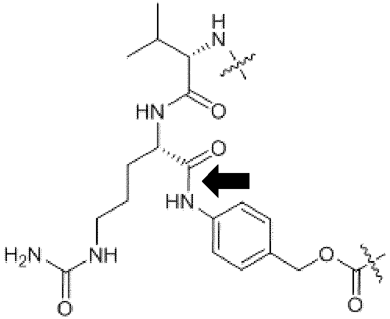

The resultant X-drug is not a stable intermediate and spontaneously undergoes elimination leaving drug as the product (*i.e*. a 1,6-elimination (self-immolation)). For more information about valine-citrulline dipeptide as an intracellular cleavage mechanism by cathepsin B, see for example Dubowchik GM, Firestone RA, Padilla L, Willner D, Hofstead SJ, Mosure K, et al. Bioconjug Chem. 2002;13:855-69.

X is an aminobenzyl ester self-immolative group bonded to Z, notably represented by formula (IV). X is linked to Z on the side of the carboxylic group (*i.e*. carbonyl function) of Z. In an embodiment, linkage between Z and X occurs between the carbonyl group of Z and the amino function of X. For more information about aminobenzyl ester self-immolative group, see WO 03/026577 or WO 2004/010957. As disclosed herein, X is therefore a bifunctional chemical moiety spacer, which (i) is capable of covalently linking together at least two other chemical moieties into a stable molecule (notably D and Z), (ii) can be released from one of the spaced chemical moieties from the molecule by means of enzymatic cleavage (after cleavage by cathepsin B, X-D is released); and (iii) following enzymatic cleavage, can spontaneously cleave from the remainder of the molecule to release the other of the spaced chemical moieties (finally D is released).

### Payloads

As used herein, the term "drug" or D in the formula (I) or (II) of the ADC also refers to "a payload", *i.e.* a moiety that is conjugated to an antibody (or a fragment).

In one embodiment, D of the disclosure is a payload which is linked to X on the side of the carbonyl function of X. In an embodiment, linkage between X and D occurs between carbonyl function of X and amino group of D.

In a specific embodiment, in ADC of the disclosure, D is a cytotoxic drug.

In an embodiment, the cytotoxic drug is selected from the group consisting of: taxon, cytochalasin B, auristatin, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, procaine, tetracaine, lidocaine, propranolol, and puromycin and analogs or homologs thereof. This includes also antimetabolites (e.g., methotrexate, 6-mercaptopurine, 6-thioguanine, cytarabine, 5-fluorouracil decarbazine), ablating agents (*e.g*., mechlorethamine, thioepa chloraxnbucil, meiphalan, carmustine (BSNU) and lomustine (CCNU), cyclothosphamide, busulfan, dibromomannitol, streptozotocin, mitomycin C, and cis-dichlorodiamine platinum (II) (DDP) cisplatin, anthracyclines (*e.g*., daunorubicin (formerly daunomycin) and doxorubicin), antibiotics (*e.g*., dactinomycin (formerly actinomycin), bleomycin, mithramycin, and anthramycin (AMC)), and anti-mitotic agents (*e.g*., vincristine and vinblastine). Preferably, the drug is monomethyl auristatin E.

### ADC of the disclosure

More preferably, the present disclosure provides the use of ADC where an anti-TfR antibody is linked to a drug (notably MMAE). Linkers are molecules L', L, Z and X as defined above. Preferably such ADC can selectively deliver an effective dose of a MMAE to tumor tissues expressing TfR.

In a preferred embodiment, the present disclosure provides ADC of formula (II) as defined above.

In a more preferred embodiment, ADC of the disclosure is as follows:
- Ab is an anti-TfR antibody, notably mAb1 as defined above,
- L is a linker molecule bonded to said antibody, said linker molecule being of formula (V),
- Z is a dipeptide of valine-citrulline bonded to L,
- X is bonded to Z and is a para-aminobenzyl ester self-immolative group of formula (IV) wherein m is 0,
- D is a drug bonded to X, notably MMAE.

In another more preferred embodiment, ADC of the disclosure called "INA03" is as follows:
- Ab is the anti-TfR antibody mAb1 characterized by a Heavy Chain represented by SEQ ID NO:18 and a Light Chain represented by SEQ ID NO:17,
- L is a linker molecule bonded to said antibody, said linker molecule being of formula (V),
- Z is a dipeptide of valine-citrulline bonded to L,
- X is bonded to Z and is a para-aminobenzyl ester self-immolative group of formula (IV) wherein m is 0,
- D is MMAE.

In an embodiment, ADC of the disclosure comprises an antibody, preferably an antibody characterized by a Heavy Chain represented by SEQ ID NO:18 and a Light Chain represented by SEQ ID NO:17, linked to L-Z-X-D of formula (X) below:

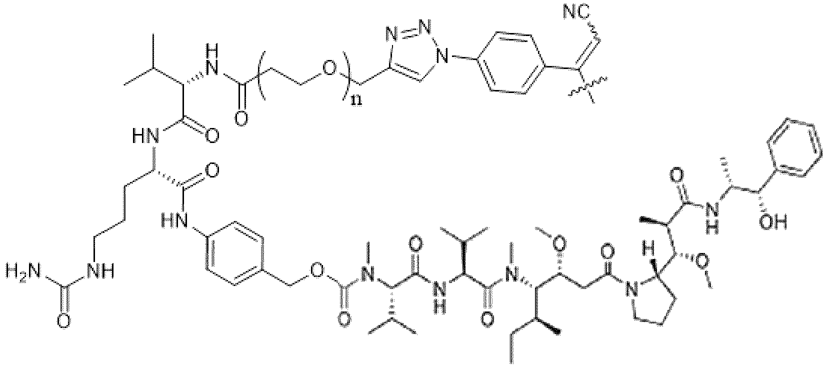

### BH3 Mimetics

The present disclosure relates to the use of an ADC in combination with at least one BH3 mimetics.

As used herein, the term "BH3 mimetics" (or "BH3 mimetic") refers to a class of compounds that compete for the hydrophobic groove to antagonize the function of anti-apoptotic proteins. BH3-only proteins promote apoptosis by both directly activating pro-apoptotic proteins and by suppressing the anti-apoptotic proteins at the mitochondria and the endoplasmic reticulum.

In an embodiment, a BH3 mimetics possess the following properties:
1. Selective induction of MOMP (Mitochondrial Outer Membrane Permeabilization) in mitochondria from cells of known dependence on the putative anti-apoptotic protein target (*e.g*., a putative MCL-1 inhibitor should selectively induce MOMP in the mitochondria from the MCL-1-dependent cell line);
2. Selective killing of cells of known dependence on the putative anti-apoptotic protein target (e.g., a putative MCL-1 inhibitor should kill the MCL-1-dependent cell line.)
3. Lack of MOMP induction in mitochondria lacking BAX and BAK, and lack of killing of cell lines lacking BAX and BAK.
(see Villalobos-Ortiz, M., Ryan, J., Mashaka, T.N. et al. BH3 profiling discriminates on-target small molecule BH3 mimetics from putative mimetics. Cell Death Differ 27, 999-1007 (2020). https://doi.org/10.1038/s41418-019-0391-9). Villalobos-Ortiz *et al.* also describe a test for screening BH3 mimetics.

In an embodiment, BH3 mimetics is chosen among:
- a BCL-2 and BCL-XL inhibitor;
- a BCL-2 inhibitor;
- a BCL-XL inhibitor; or
- a MCL-1 inhibitor.

For example, a BH3 mimics is chosen among the following compounds:
- BCL-2 and BCL-XL inhibitors: ABT-737 (*e.g*. CAS number 852808-04-9), ABT-263 (*e.g*. CAS number 923564-51-6) and AZD4320 (*e.g*. CAS number 1357576-48-7);
- BCL-2 inhibitor: ABT-199 (*e.g.* Cas number 1257044-40-8);
- BCL-XL inhibitors: WEHI-539 (*e.g.* Cas number 1431866-33-9), A-1155463 (*e.g*. Cas number 1235034-55-5) and A-1331852 (*e.g.* Cas number: 1430844-80-6); and
- MCL-1 inhibitors: A-1210477 *(e.g.* Cas number: 1668553-26-1), S63845 (*e.g*. Cas number: 1799633-27-4), AMG176 (*e.g*. Cas number: 1883727-34-1), S64315 (*e.g*. Cas number: 1799631-75-6), and AZD5991 (*e.g*. Cas number: 2143061-81-6).

Preferably, BH3 mimetics are venetoclax, navitoclax, obatoclax, even more preferably venetoclax.

Venetoclax (DCI), also called ABT-199, selectively binds and inhibits the B-cell lymphoma-2 (BCL-2) protein. In some blood cancers, BCL-2 prevents cancer cells from undergoing apoptosis. IUPAC name of Venetoclax is 4-[4-[[2-(4-chlorophenyl)-4,4-dimethylcyclohexen-1-yl]methyl]piperazin-1-yl]-*N-*[3-nitro-4-(oxan-4-ylmethylamino)phenyl]sulfonyl-2-(1*H*-pyrrolo[2,3-b]pyridin-5-yloxy)benzamide.

Navitoclax, also called ABT-263, selectively binds to apoptosis suppressor proteins Bcl-2, Bcl-XL, and Bcl-w and prevents their binding to the apoptotic effectors Bax and Bak proteins. IUPAC name of Navoticlax is 4-[4-[[2-(4-chlorophenyl)-5,5-dimethylcyclohexen-1-yl]methyl]piperazin-1-yl]-N-[4-[[(2*R*)-4-morpholin-4-yl-1-phenylsulfanylbutan-2-yl]amino]-3-(trifluoromethylsulfonyl)phenyl]sulfonylbenzamide.

Obatoclax, also called GX15-070, is a pan-inhibitor of Bcl-2 family proteins, with pro-apoptotic activity. It is a selective antagonist of the BH3-binding groove of the Bcl-2 family proteins, which are overexpressed in some cancers. IUPAC name of Obatoclax is (2*Z*)-2-[(5*Z*)-5-[(3,5-dimethyl-1*H*-pyrrol-2-yl)methylidene]-4-methoxypyrrol-2-ylidene]indole.

### Use of the ADC/Method of treatment

The disclosure notably relates to antibody-drug conjugate of the formula (I) or of the formula (II) as defined above, for use in the treatment of a tumor, characterized in that said antibody-drug conjugate is administered in combination with at least one BH3 mimetics compound.

The disclosure also relates to a method for the treatment of a tumor, comprising the steps of:
- administration of a therapeutically efficient amount of an antibody-drug conjugate of the formula (I) or of the formula (II),
- administration of a therapeutically efficient amount of a BH3 mimetics.

In an embodiment, the combined use of ADC of the disclosure and BH3 mimetics is particularly advantageous due to additive effect, even synergic effect,

Antibody-drug conjugate of the formula (I) or of the formula (II) and at least one BH3 mimetics are administered to a subject in need thereof. In a preferred embodiment, such ADC is INA03 as defined above.

Preferably, in said use or method, ADC is of the formula (II) (such as INA03) and said BH3 mimetics is venetoclax.

Preferably, in said use or method, ADC is of the formula (II) (such as INA03) and said BH3 mimetics is navitoclax.

Preferably, in said method, ADC is of the formula (II) (such as INA03) and said BH3 mimetics is navitoclax.

Preferably, in said method, ADC is of the formula (II) (such as INA03) and said BH3 mimetics is obatoclax.

As used herein, the term "treat" "treating" or "treatment" refers to one or more of (1) inhibiting the disease; for example, inhibiting a disease, condition or disorder in an individual who is experiencing or displaying the pathology or symptomatology of the disease, condition or disorder (i.e., arresting further development of the pathology and/or symptomatology); and (2) ameliorating the disease; for example, ameliorating a disease, condition or disorder in an individual who is experiencing or displaying the pathology or symptomatology of the disease, condition or disorder (i.e., reversing the pathology and/or symptomatology) such as decreasing the severity of disease or reducing or alleviating one or more symptoms of the disease. In particular, with reference to the treatment of a tumor, the term "treatment" may refer to the inhibition of the growth of the tumor, or the reduction of the size of the tumor. Preferably, said tumor is a solid tumor or a hematologic tumor, and more specifically a lymphoma or leukemia. More preferably, the antibody-drug conjugate according to the disclosure is for use for the treatment of venetoclax-resistant lymphoma or venetoclax-resistant leukemia.

As used herein, a "therapeutically efficient amount" or "effective amount" of an ADC is an amount sufficient to perform a specifically stated purpose, for example to product a therapeutic effect after administration, such as inhibition or reduction in tumor growth rate or tumor volume, a reduction in a symptom of cancer or some indicia of treatment efficacy. In the case of cancer, a therapeutically effective amount of ADC can reduce the number of cancer cells, reduce the tumor size, inhibit (e.g. slow or stop) tumor metastasis, inhibit (*e.g*. slow or stop) tumor growth, and/or relieve one or more symptoms.

According to the disclosure, said antibody-drug conjugate and BH3 mimetics are administered simultaneous, separate or spread over time.

As used herein, a simultaneous administration means that antibody-drug conjugate and BH3 mimetics are administered *per* the same track and at the same time or substantially at the same time.

As used herein, a separate administration means that antibody-drug conjugate and BH3 mimetics, are administered at the same time or substantially at the same time by different ways.

As used herein, an administration spread over time, means that antibody-drug conjugate and BH3 mimetics are administered at different times, the route of administration being identical or different. More particularly, it can mean the entire administration of one of the active ingredients before the beginning of administration of the other(s). Preferably said antibody-drug conjugate is administered intravenously and said BH3 mimetics are administered orally.

### Compositions

In another aspect, the present disclosure provides a composition, *e.g*. a pharmaceutical composition, containing one or a combination of ADC disclosed herein with a BH3 mimetics. The present disclosure also provides a composition containing an ADC of the disclosure and another composition containing a BH3 mimetics. A pharmaceutical composition comprises a pharmaceutically acceptable carrier.

As used herein, "pharmaceutically acceptable carrier" includes any and all solvents, dispersion media, coatings, antibacterial and antifungal agents, isotonic and absorption delaying agents, and the like that are physiologically compatible. The pharmaceutical formulation of the disclosure may further comprise one or more pharmaceutically acceptable excipients selected from stabilizers, surfactants, buffering agents, antimicrobial preservatives, protectants, antioxidants, chelating agents, bulking agents.

The form of the pharmaceutical compositions, the route of administration, the dosage and the regimen naturally depend upon the condition to be treated, the severity of the illness, the age, weight, and sex of the patient.

The pharmaceutical compositions of the disclosure can be formulated for a topical, oral, parenteral, intraperitoneal, intranasal, intravenous, intramuscular, subcutaneous or intraocular administration and the like, preferably intraperitoneal or intravenous.

Preferably, the pharmaceutical compositions contain vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

To prepare pharmaceutical compositions, an effective amount of the ADC may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders or lyophilisates for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

An ADC of the disclosure can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

The invention having been fully described is now further illustrated by the following examples, which are illustrative only and are not meant to be further limiting.

### EXAMPLES

### Example 1. Combination effect on cellular proliferation of INA03 and Venetoclax

Cellular proliferation/viability of several cell lines was tested in presence of Venetoclax alone or in combination with INA03:
Cells are cultured in RPMI medium supplemented with Fetal Bovine serum (10%).

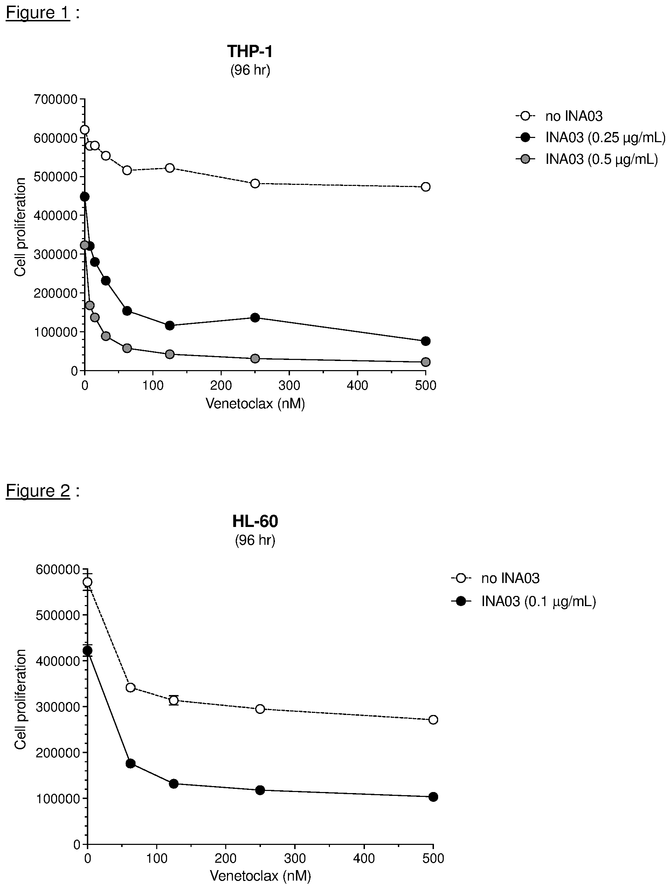

The AML cell line THP-1 (resistant to Venetoclax) was treated by several doses of Venetoclax alone or in combination with INA03 (at 0.25 or 0.5 µg/mL) for 96h. The results obtained at 96h are represented in **Figure 1****.**

The AML cell line HL-60 (sensitive to Venetoclax) was treated by several doses of Venetoclax alone or in combination with INA03 (at 0.1 µ/mL) for 96h. The results obtained at 96h are represented in **Figure 2****.**

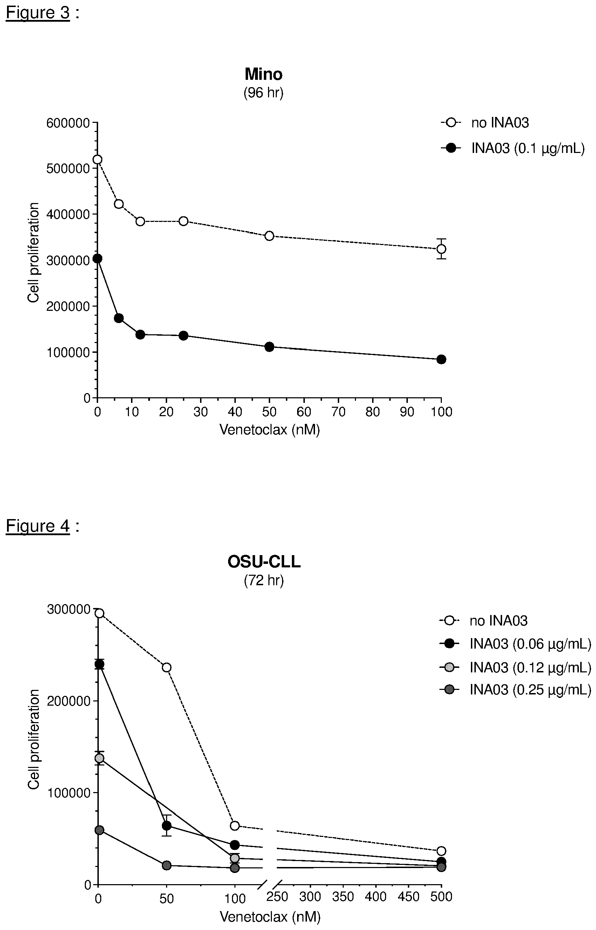

The Mantle cell lymphoma line Mino was treated by several doses of Venetoclax alone or in combination with INA03 (at 0.1 µ/mL) for 96h. The results obtained at 96h are represented in **Figure 3****.**

The CLL cell line OSU was treated by several doses of Venetoclax alone or in combination with INA03 (at 0.06, 0.12 or 0.25 µg/mL) for 72h. The results obtained at 72h are represented in **Figure 4****.**

In all tests, the cell viability was measured by using the Cell Titer-Glo^{®}-Luminescent Cell Viability Assay estimating the ATP content (Promega).

### Example 2. Combination effect of INA03 and Venetoclax on apoptosis of primary AML cells

AML cells (M4 or M5 subtype) were treated with several doses of Venetoclax alone or in combination with INA03 (at 0.1 or 0.25 µg/mL) for 96 h.

Percentage of apoptotic cells was determined by flow cytometry has Annexin-V / PI double positive cells.

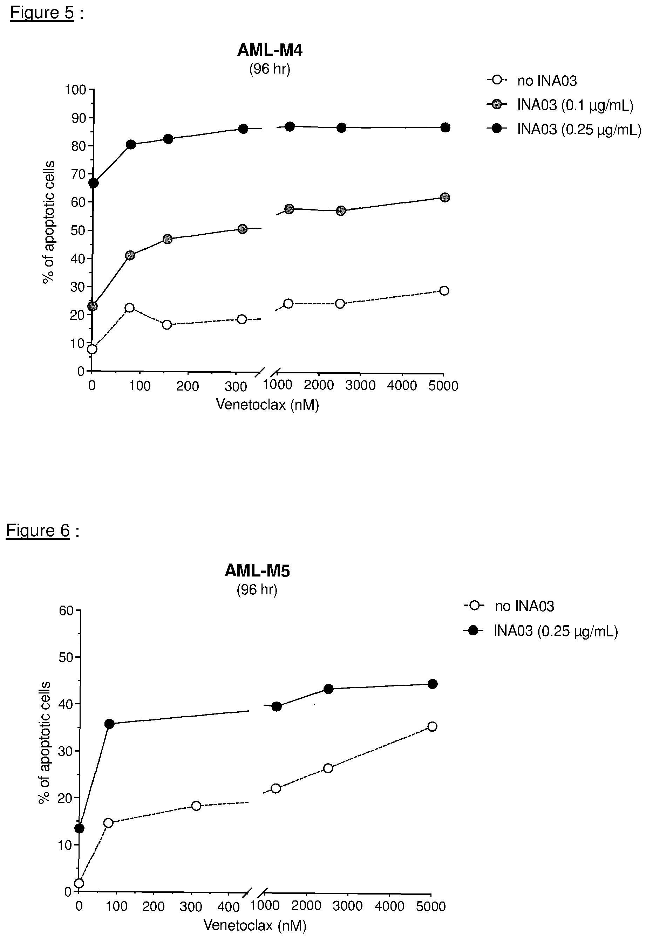

The results obtained at 96h are represented in **Figures 5** and **6****.**

### Example 3. Time course of proliferation on THP-1 cell

Time course of proliferation on THP-1 cell in the presence of INA03 alone (1 µg/mL), Venetoclax (200 nM) alone or in combination of both INA03 and Venetoclax (respectively 1 µg/mL and 200 nM) was analyzed during 60 hours.

Cells were plated in poly-L-ornithine treated cell culture plate. 10 000 cells per well are added in 0.1 mL. 100 ul of Annexin V-Red or Caspase 3/7 green were added, then the plate is put back in the incubator. In the incubator there is a Incucyte^{®} plate reader that takes fluorescence every 3 hours.

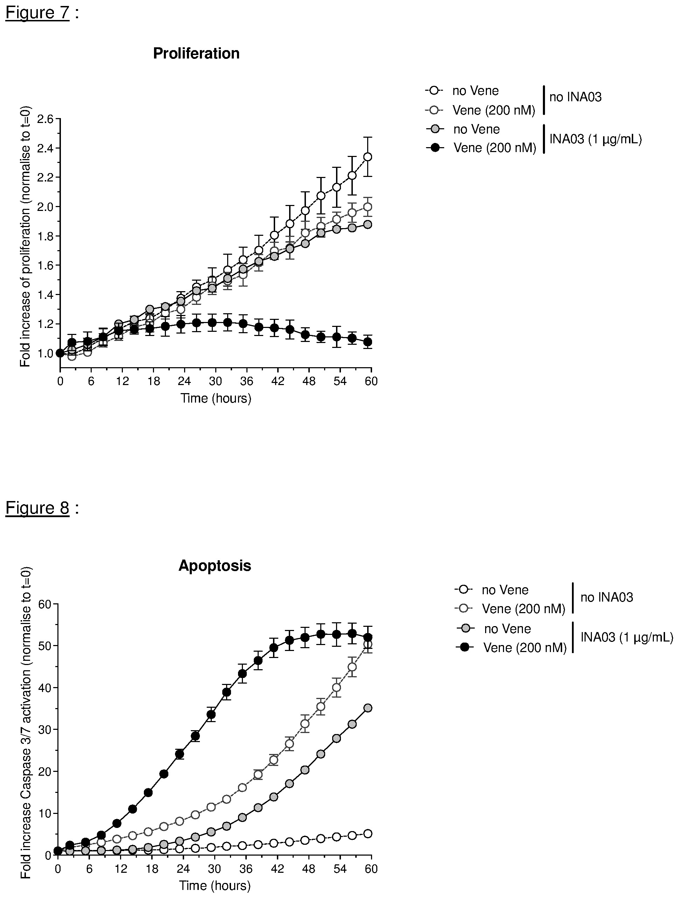

The results are represented in **Figure 7****.**

### Example 4. Time course of apoptosis on THP-1 cell

Time course of apoptosis on THP-1 cell in the presence of INA03 alone (1 µg/mL), Venetoclax (200 nM) alone or in combination of both INA03 and Venetoclax (respectively 1 µg/mL and 200 nM). Apoptosis has been quantified as the number of green fluorescent caspase-3/7 active objects for each time point.

Method is the same as in Example 3.

The results are represented in **Figure 8****.**

### Example 5. Analysis of mitochondrial membrane potential and caspase 8 positive cells.

The percentage of cells with depolarized mitochondria and caspase 8 positive cells (which illustrates the cellular death) was analyzed.

THP-1 cells were incubated for 24h in medium culture alone (ctrl) or in the presence of INA03 (1 ug/mL), Venetoclax (200 nM) or a combination of both at the same dose.

Medium culture comprises a mitochondrial marker DiIC1(5) or caspase 8 antibody, at room temperature (10⁶ cells/mL in 0.3 mL of medium culture). Cells are centrifugated, then incubated in a buffer comprising annexin V at 37°C during 15 minutes and analyzed by flow cytometry.

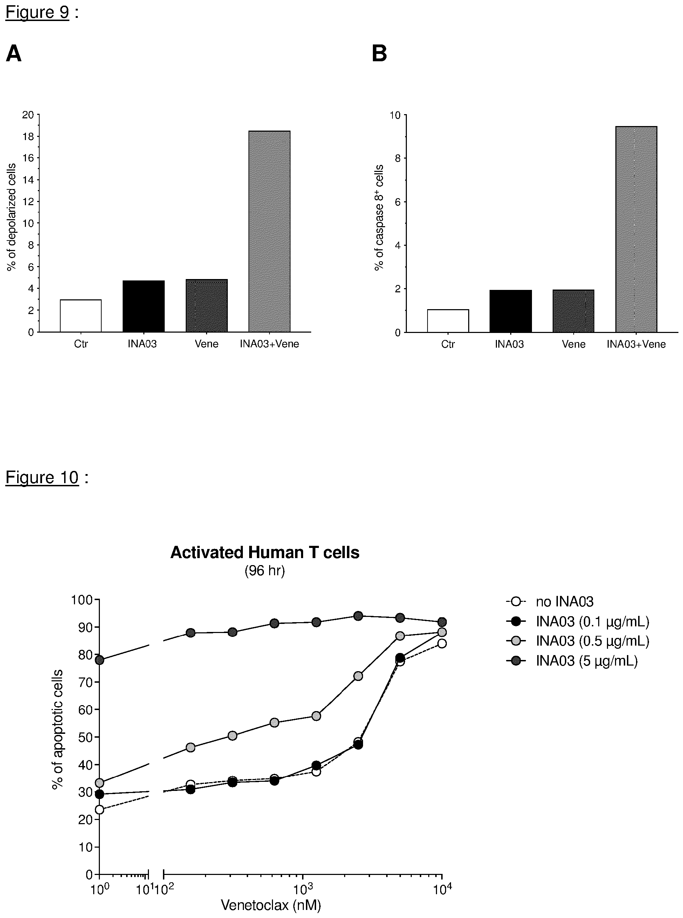

The results are represented in **Figure 9A** and 9B

### Example 6. Apoptosis on activated and non-activated T cells

T cells were incubated for 96h in the presence of INA03 and Venetoclax alone or in combination, at various concentrations. The percentage of apoptosis on activated and non-activated T cells is then calculated. PHA and IL-2 are also added into the culture medium.

The results are represented in **Figures 10** (activated cells) and **11** (non-activated cells).

### Example 7. Combination effect on cellular proliferation of INA03 and Navitoclax

Cells are cultured in RPMI medium supplemented with Fetal Bovine serum (10%).

The AML cell line THP-1 was treated by several doses of Navitoclax alone or in combination with INA03 (at 0.25 or 0.5 µg/mL). The cell viability was measured by using the Cell Titer-Glo^{®}-Luminescent Cell Viability Assay estimating the ATP content (Promega).

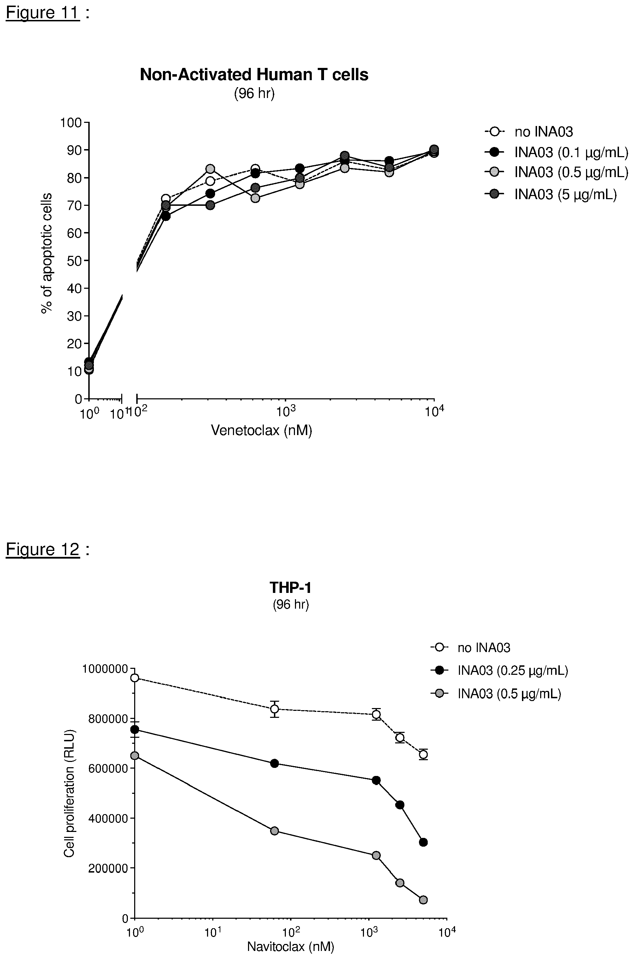

The results are represented in **Figure 12****.**

### Example 8. Combination effect on cellular proliferation of INA03 and Obatoclax

Cells are cultured in RPMI medium supplemented with Fetal Bovine serum (10%).

The AML cell line THP-1 was treated by several doses of Obatoclax alone or in combination with INA03 (at 0.25 or 0.5 µg/mL). The cell viability was measured by using the Cell Titer-Glo^{®}-Luminescent Cell Viability Assay estimating the ATP content (Promega).

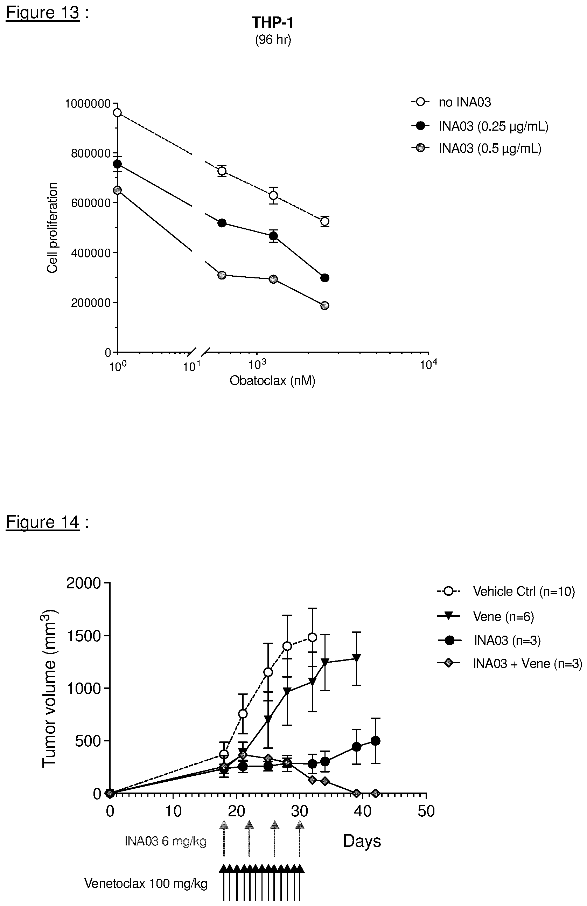

The results are represented in **Figure 13****.**

### Example 9. In vivo efficacy of INA03 in combination with Venetoclax

To assess the *in vivo* efficacy of the INA03 in combination with the BH3-mimetic Venetoclax, nude mice were injected subcutaneously with 1x10⁶ HL-60 cells. When tumors reach 100 mm³, mice were injected in a curative mode with, either 6 mg/kg (q4dx4) intravenously, alone (closed circles) or 13 consecutive days oral gavage with Venetoclax at 100 mg/kg (closed triangles) or both (grey diamonds). Animals were also injected intravenously with the vehicle as controls (open circles). Tumor growths were monitored every day and mice were sacrificed when the tumors reach around 1500 mm³.

The results are represented in **Figure 14** and represent the tumor growth of the mice in days, after multiple injections of INA03 with or without Venetoclax.

## Claims

1. An antibody-drug conjugate of the formula (I): Ab-[L'-D]p (I), wherein:
- Ab is an anti-Transferrin Receptor (TfR) antibody which binds specifically to SEQ ID NO:16,
- L' is a cleavable or a non-cleavable linker moiety covalently bonded to said antibody, preferably a cleavable linker,
- D is a cytotoxic drug moiety covalently bonded to L',
- p is an integer from 1 to 8, preferably 4,
for use in the treatment of a tumor, **characterized in that** said antibody-drug conjugate is administered in combination with at least one BH3 mimetics compound.

2. An antibody-drug conjugate for use according to claim 1, wherein said antibody-drug conjugate is of the formula (II): Ab-L-Z-X-D, wherein:
- Ab is an anti-TfR antibody,
- L is a linker molecule bonded to said antibody, said linker molecule being of formula (III): 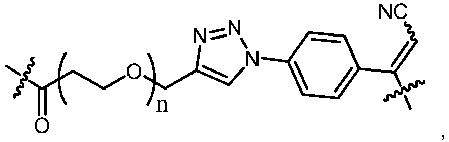 wherein n is an integer comprised between 2 and 20,
- Z is a dipeptide of valine-citrulline bonded to L,
- X is an aminobenzyl ester self-immolative group bounded to Z,
- D is a drug bounded to X.

3. The antibody-drug conjugate for use according to any one of claims 1-2, wherein said BH3 mimetics is chosen among: venetoclax, navitoclax, obatoclax and S64315, preferably venetoclax, navitoclax, obatoclax, more preferably venetoclax.

4. The antibody-drug conjugate for use according to any one of claims 1-3, wherein the drug is a cytotoxic drug, notably monomethyl auristatin E.

5. The antibody-drug conjugate for use according to any one of claims 1-4, wherein X is a para-aminobenzyl ester group covalently bonded to Z, said X being of the following formula (IV): 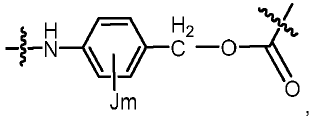 J being an optional substituent chosen among F, Cl, Br, NO₂, NHCOCH₃, N(CH₃)₂, NHCOCF₃, alkyl, and haloalkyl, and m being an integer of 0, 1, 2, 3 and 4,
said X being preferably a para-aminobenzyl ester wherein m is 0.

6. The antibody-drug conjugate for use according to any one of claims 1-5, wherein L is covalently bonded to one or more thiol residues of said antibody,
preferably, said L corresponding to a linker molecule of formula (V):

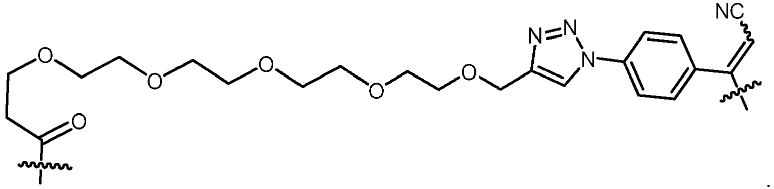

7. The antibody-drug conjugate for use according to any one of claims 1-6, wherein said antibody includes full-length antibodies or antibodies fragments containing antigen binding portions.

8. The antibody-drug conjugate for use according to any one of claims 1-7, wherein said anti-TfR antibody comprises either:
(a) a variable heavy chain polypeptide comprising HCDR1 of SEQ ID NO:1, HCDR2 of SEQ ID NO:2, HCDR3 of SEQ ID NO:3 and a variable light chain polypeptide comprising LCDR1 of SEQ ID NO:4, LCDR2 of SEQ ID NO:5 and LCDR3 of SEQ ID NO:6;
(b) a variable heavy chain polypeptide comprising HCDR1 of SEQ ID NO:1, HCDR2 of SEQ ID NO:2, HCDR3 of SEQ ID NO:3 and a variable light chain polypeptide comprising LCDR1 of SEQ ID NO:4, LCDR2 of SEQ ID NO:8 and LCDR3 of SEQ ID NO:6;
(c) a variable heavy chain polypeptide comprising VH of SEQ ID NO:11 and a variable light chain polypeptide comprising VL of SEQ ID NO:13;
(d) a variable heavy chain polypeptide comprising VH of SEQ ID NO:11 and a variable light chain polypeptide comprising VL of SEQ ID NO:14;
(e) a variable heavy chain polypeptide comprising VH of SEQ ID NO:11 and a variable light chain polypeptide comprising VL of SEQ ID NO:15;
(f) a variable heavy chain polypeptide comprising VH of SEQ ID NO:12 and a variable light chain polypeptide comprising VL of SEQ ID NO:13;
(g) a variable heavy chain polypeptide comprising VH of SEQ ID NO:12 and a variable light chain polypeptide comprising VL of SEQ ID NO:14;
(h) a variable heavy chain polypeptide comprising VH of SEQ ID NO:12 and a variable light chain polypeptide comprising VL of SEQ ID NO:15,
preferably said antibody is mAb1 comprising heavy chain of SEQ ID NO:18 and light chain of SEQ ID NO:17.

9. The antibody-drug conjugate for use according to any one of claims 1-8, wherein:
said antibody-drug conjugate is of the formula (II): Ab-L-Z-X-D, wherein:
- Ab is an antibody comprising heavy chain of SEQ ID NO:18 and light chain of SEQ ID NO:17,
- L is a linker molecule of formula (V),
- Z is a dipeptide of valine-citrulline,
- X is a para-aminobenzyl ester of formula (III) wherein m is 0,
- D represents monomethyl auristatin E, and
wherein BH3 mimetics is venetoclax, navitoclax or obatoclax, preferable venetoclax.

10. The antibody-drug conjugate for use according to any one of claims 1-8, wherein said tumor is a solid tumor or a hematologic tumor, and more specifically a lymphoma or leukemia.

11. The antibody-drug conjugate for use according to any one of claims 1-10, for the treatment of venetoclax-resistant lymphoma or venetoclax-resistant leukemia.

12. A BH3 mimetics compound for use in the treatment of a tumor, **characterized in that** said BH3 mimetics compound is administered in combination with at least antibody-drug conjugate of the formula (I): Ab-[L'-D]p (I), wherein:
- Ab is an anti-Transferrin Receptor (TfR) antibody which binds specifically to SEQ ID NO:16,
- L' is a cleavable linker moiety covalently bonded to said antibody,
- D is a cytotoxic drug moiety covalently bonded to L',
p is an integer from 1 to 8, preferably 4.

13. A composition comprising:
(a) an antibody-drug conjugate of the formula (I): Ab-[L'-D]p (I), wherein:
- Ab is an anti-Transferrin Receptor (TfR) antibody which binds specifically to SEQ ID NO:16,
- L' is a cleavable linker moiety covalently bonded to said antibody,
- D is a cytotoxic drug moiety covalently bonded to L',
p is an integer from 1 to 8, preferably 4,
(b) a BH3 mimetics, and
(c) one or more pharmaceutical acceptable excipient, diluent or carrier.

14. A combination product comprising:
(a) an antibody-drug conjugate of the formula (I): Ab-[L'-D]p (I), wherein:
- Ab is an anti-Transferrin Receptor (TfR) antibody which binds specifically to SEQ ID NO:16,
- L' is a cleavable linker moiety covalently bonded to said antibody,
- D is a cytotoxic drug moiety covalently bonded to L',
p is an integer from 1 to 8, preferably 4, and
(b) a BH3 mimetics.

15. A composition according to claim 13 or a combination product according to 14, for use in the treatment of a tumor.
